(19) Europäisches Patentamt
European Patent Office
Office européen des brevets

(11) EP 0 565 607 B1

(12) **FASCICULE DE BREVET EUROPEEN**

(45) Date de publication et mention de la délivrance du brevet:
**21.04.1999 Bulletin 1999/16**

(21) Numéro de dépôt: **92903453.6**

(22) Date de dépôt: **31.12.1991**

(51) Int Cl.[6]: **C07K 1/06**, C07K 1/14, C07K 1/04

(86) Numéro de dépôt international:
**PCT/FR91/01083**

(87) Numéro de publication internationale:
**WO 92/12167 (23.07.1992 Gazette 1992/19)**

# (54) GROUPE PROTECTEUR, COMPOSE PROTEGE PAR CE GROUPE ET DISPOSITIF POUR GREFFER LE GROUPE PROTECTEUR SUR LE COMPOSE A PROTEGER

SCHUTZGRUPPE, DURCH DIESE GRUPPE GESCHÜTZTE VERBINDUNG, UND VORRICHTUNG ZUM ANBRINGEN DER SCHUTZGRUPPE AN DIE ZU SCHÜTZENDEN VERBINDUNG

PROTECTION GROUP, COMPOUD PROTECTED BY THIS GROUP AND DEVICE FOR GRAFTING THE PROTECTION GROUP ONTO THE COMPOUND BEING PROTECTED

(84) Etats contractants désignés:
**AT BE CH DE DK ES FR GB GR IT LI LU NL SE**

(30) Priorité: **31.12.1990 GB 9028208**

(43) Date de publication de la demande:
**20.10.1993 Bulletin 1993/42**

(73) Titulaire: **RHODIA CHIMIE**
**92408 Courbevoie Cédex (FR)**

(72) Inventeurs:
- **RAPHY, Gilles**
  **F-74000 Annecy-le-Vieux (FR)**
- **RAMAGE, Robert**
  **Edinburgh RH4 3JZ (GB)**

(74) Mandataire: **Ricalens, François et al**
**RHODIA CHIMIE**
**Direction de la Propriéte Industrielle**
**25, quai Paul Doumer**
**92408 Courbevoie Cédex (FR)**

(56) Documents cités:
- **SCIENCE, vol. 232, 18 avril 1986; B. MERRIFIELD, pp. 341-347**
- **CHEMICAL COMMUNICATIONS, 1968; J.W. WILSON et al., p. 454**
- **SYNTHESIS (Papers), May 1986; A.C. HOPKINSON et al., pp. 366-371**
- **CHEMICAL ABSTRACTS, 12th Collective Index, pp. 78778CS-78781CS**
- **C.A., 12 th coll index, p. 78778 CS à 78781 CS.**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Jouve, 75001 PARIS (FR)

## Description

La présente invention se rapporte à un nouveau groupe protecteur et à son utilisation, en particulier dans la synthèse des peptides. Elle se rapporte plus particulièrement à un groupe protecteur qui facilite la purification des composés, surtout les peptides, pendant ou à la fin d'une synthèse.

En synthèse organique, en particulier multi-étapes, la purification des produits obtenus peut poser plus de problèmes que la synthèse elle-même. Ceci est particulièrement vrai dans le cas de la synthèse des peptides, une synthèse qui utilise systématiquement les groupes protecteurs et qui peut également jouer des rôles supplémentaires.

Ainsi, le besoin de protéger, ou d'activer une certaine fonction dans les acides aminés a été utilisé pour réaliser des synthèses de peptides en biphase afin de faciliter les stades de purification. Cependant, le problème de la purification dans la synthèse des peptides (la problématique est parfaitement exposée dans l'indrodution de l'article publié par Bruce Merrifield dans Science (1986) volume 232, page 341 à 347,notamment le passage intitulé "synthesis on a solid matrix"), implicite dans les techniques telles que la technique Merrifield (J. Amer Chem. Soc. 1986, 108, 5242), n'a pas été complètement résolu.

Une des solutions les plus élégantes à ce problème serait de modifier le peptide, ou toute autre molécule nécessitant une protection, en le liant à un solide d'une façon physiquement réversible, au cours de l'élaboration et de la purification. A ce jour, aucun groupe protecteur n'a été révélé comme étant capable d'exercer une telle propriété. C'est pour cette raison qu'un des objectifs principaux de la présente invention est de fournir un groupe protecteur correspondant aux critères ci-dessus.

Le brevet français N°2.146.044 vise un procédé de protection au moyen du groupe fluorényle mais ne présente pas les propriétés souhaitables ci dessus.

La demande de brevet européen 0287882 vise un procédé de synthèses peptitique en utilisant comme support une résine acide mais très limité dans son application car ne permettant ni l'utilisation d'acide ni l'utilisation de base forte.

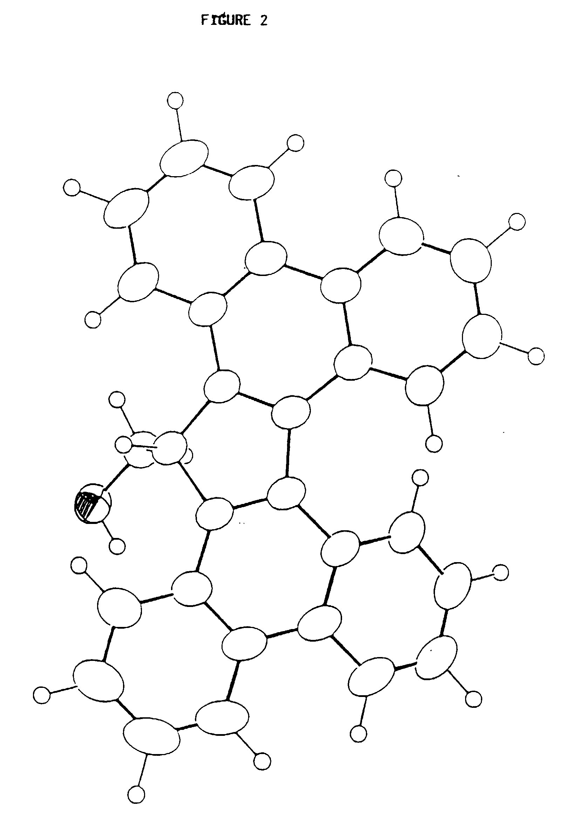

Les Figures 1 et 2 montrent la structure radiographique du cristal d'un des composés de la présente invention, conformément aux descriptions dans les Exemples. **Figure 1** : structure de cristal radiographique, (a) vue "latérale" de (31) montrant la cellule de l'unité. **Figure 2 :** structure de cristal radiographique, (b) vue "de dessus" de (31) ;

La présente invention fournit un procédé caractérisé par le fait qu'il comporte l'étape de lier physiquement à un matériau carboné une molécule dont au moins une fonction est protégée par un groupe de formule (I):

Ar-L-

où

Ar représente un système de cycles fusionnés substantiellement plan contenant au moins 4 cycles aromatiques, et L représente un groupe contenant au moins un atome de carbone qui est capable de se lier à une fonction à protéger; et

où

ledit matériau carboné est choisi parmi le graphite ou ceux à surfaces graphitisées y compris le charbon activé .

Le groupe Ar contient de préférence au moins 6 cycles aromatiques et les cycles aromatiques sont de préférence hexagonaux. De préférence, Ar ne contient pas d'hétéro-atome.

Le groupe L peut être, par exemple, un groupe d'alcoyle ou, en particulier, un groupe de peptide. En général, le groupe L peut être composé de tout cycle, ou branche, dont les spécialistes savent qu'il ou elle forme un lien entre le groupe protecteur et la molécule à protéger. Le groupe L est de préférence relié au groupe Ar par un atome de carbone. Le groupe L est choisi de façon à ce qu'il n'y ait pas de conjugaison entre le groupe Ar et le groupe partant. De préférence, le groupe L comprend le groupe - CR'-L'-, où L' est défini comme ci-dessous et R' est un groupe alcoyle, ayant de préférence jusqu'à 4 atomes de carbone, ou d'hydrogène.

Le groupe idéal de la présente invention présente la formule (II) :

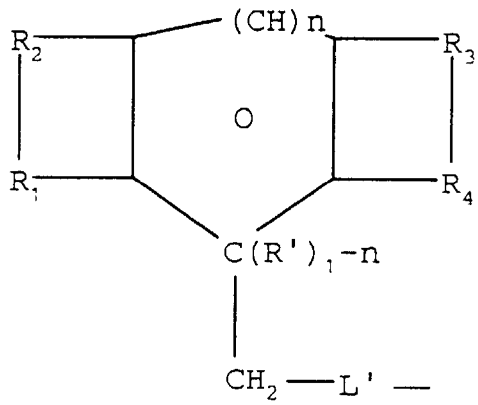

où

n est égal à 0 ou 1;
O signifie que le cycle environnant est aromatique quand n est égal à 1 et non-aromatique quand n est égal à 0 ;
R1 et R2, d'une part, et R3 et R4, d'autre part, sont choisis de façon à ce que a chacun forme un système de cycles aromatiques fusionnés ensemble avec les atomes de carbone auxquels ils sont rattachés ;
R' est un groupe alcoyle ou hydrogène ; et L' représente un lien direct ou un groupe capable de se lier à un groupe à protéger.

Pour rigidifier le groupe protecteur, il est possible de prévoir un groupe R5 choisi de façon à former un ou plusieurs cycles aromatiques, les cycles étant de préférence de structure hexagonale ne contenant avantageusement aucun hétéro-atome, pour donner une formule de groupe (III) :

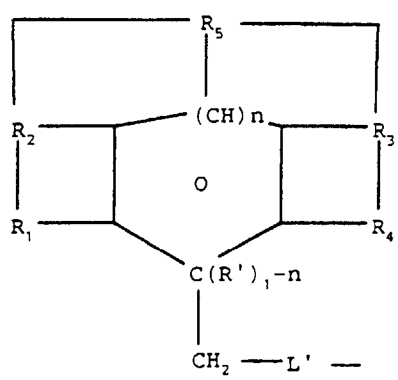

Quand n est égal à 0, il n'y a aucun lien entre le cycle central et R5 et quand n est égal à un le lien entre R5 et R3 est optionnel mais préférable.

Le nombre total de cycles aromatiques se situe avantageusement de 4 à 8. Les apogées libres peuvent être remplacées par des groupes alcoyles "lato sensu", ayant avantageusement une chaîne courte (de 1 à 5 atomes de carbone). Le nombre total d'atomes de carbone (sans tenir compte de l'anneau L) est généralement de 20 à 60, de préférence de 25 à 50.

Pour des raisons de facilité d'accès à la synthèse, R1 et R2, d'une part, R3 et R4, d'autre part, et R5 sont choisis avantageusement pour former un système qui est symétrique par rapport à l'anneau central (par rapport à la bissectrice de l'angle intra-anneau du carbone transportant le méthylène rattaché à l'anneau L).

La présente invention est basée sur le fait que l'on a découvert que les molécules protégées par le groupe défini ci-dessus absorbent très fortement le graphite ou les surfaces graphitisées y compris le charbon activé. De plus, comme nous l'expliquerons dans la partie suivante de cette description, cette absorption est réversible).

Plus il y a de cycles aromatiques transportés par le groupe, meilleure est l'absorption et plus l'élution est difficile, et vice-versa. L'optimum peut être défini cas par cas par les spécialistes. Cependant, un nombre de 4 à 8 cycles aromatiques ajoutés au cycle central constitue en général un compromis satisfaisant à la fois d'un point de vue

économique et d'un point de vue des critères d'absorption et d'élution.Les groupes où n est égal à zéro, en particulier ceux sans radical R5, sont plus facilement accessibles.

La chimie des groupes selon l'invention est la même que pour le groupe fmoc quand n est égal à zéro et la même que pour le groupe benzyle dans les autres cas, par exemple quand n est égal à un.

La chimie du groupe fmoc est bien connue et on peut se référer aux articles suivants ainsi qu'aux publications générales sur les groupes protecteurs :

CARPINO - J. Amer. Chem. Soc. 1970, 92, 5748, J. Org. Chem. 1972, 37, 3404, Synthèse 1983, p. 671, F. Org. Chem. 1983, 48, 77, Int. J. Peptide Protein Res. 1983, 22, 125, Biopolymères 1983, 22, 2157.

Les groupes ci-dessus sont faciles à obtenir par la séquence de réactions suivantes ou équivalents :

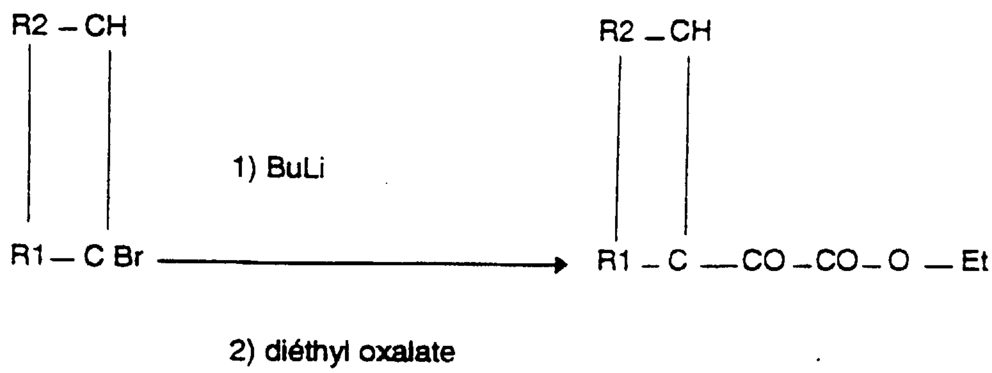


(1)

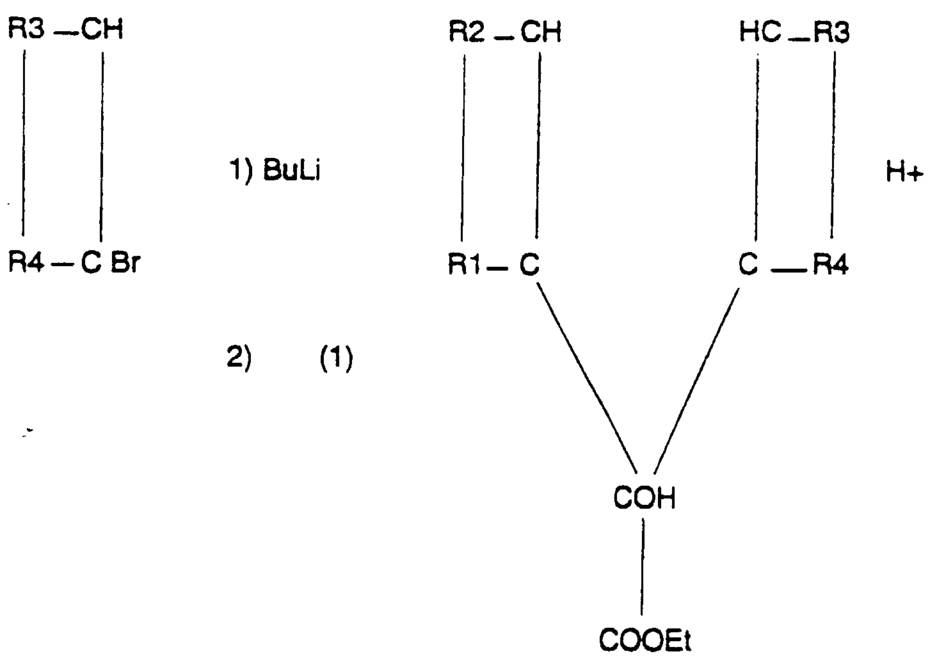

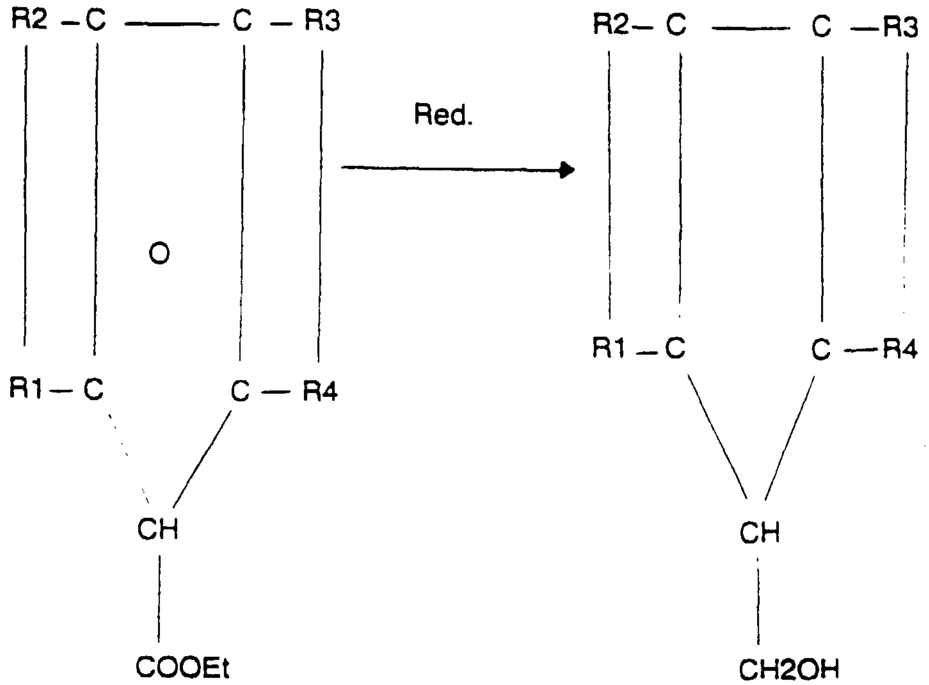


La chimie du groupe benzyle est aussi bien connue et il est approprié de se reporter aux publications spécialisées et destinées à l'enseignement sur le sujet. On peut également se référer aux articles suivants qui ont ouvert de nouvelles voies : Int. J. Peptide Protein, Res. 1986, 27, 358 ; Synthèse, p.303 ; J. Org. Chem. 1983, 48, 661 et 666. Les groupes de la présente invention peuvent être préparés selon les méthodes décrites dans les articles ci-dessus et par des techniques bien connues comme les réactions de Freidel-Crafts. Les articles ci-dessus indiquent aux spécialistes comment greffer les groupes spécifiés ci-dessus sur les fonctions à protéger. Ces groupes modifient ainsi les molécules ayant, par exemple, une fonction acide, alcool, thiol, aminée ou amidée, dont les molécules protégées sont aussi soumises à la présente application. Les molécules protégées de cette façon peuvent être des molécules de tout type, mais sont plus généralement des acides aminés ou des peptides. Les acides aminés consituants des peptides peuvent être naturels ou obtenus par synthèse ; les peptides et/ou les acides aminés peuvent être protégés ou substitués sur leurs diverses fonctions.
Selon la présente invention, il a été démontré de façon surprenante que les molécules modifiées conformément aux groupes protecteurs ci-dessus avaient la caractéristique d'être remarquablement bien retenues sur les colonnes de graphite ou sur les colonnes de matériaux graphitisés y compris le charbon activé.Ces molécules modifiées présentent également une bonne aptitude à la séparation chirale.
Il est opportun d'inclure sous le terme matériaux graphitisés ceux qui sont carbonacés totalement ou en surface et ont subi un traitement de graphitisation, en général par pyrolyse.
La forte affinité du groupe protecteur de la présente invention signifie qu'il est particulièrement adapté à la purification des peptides. L'utilisation de charbon activé offre une liaison équivalente à celle du graphite et est préférable, puisque le charbon activé est moins cher que le carbone graphitisé (PGC).
La présente invention prévoit également un composé protecteur comportant un groupe comme défini ci-dessus rattaché, par le groupe L ou L', à un groupe partant, comme un groupe qui contient un atome de azote, d'oxygène, de soufre, de sélénium, de tellure, de silicium ou d'halogène. Les exemples de groupes partants sont les groupes à atomes d'halogène (par exemple F, C1, Br ou I) ou -OH, -COOH,NH2, -CONH2, -S03C6H4-pCH3, -SH, -CN ou -Si (CH3)3.
Les groupes d'après l'invention disposent d'une autre caractéristique particulièrement avantageuse, c'est-à-dire que la grande majorité d'entre-eux absorbe les radiations visibles ou ultraviolet en longueurs d'onde qui diffèrent de celles des acides aminés naturels. Certains de ces groupes sont fluorescents à des longueurs d'onde différentes de celles des acides aminés naturels.
Cette propriété permet de développer des appareils comprenant, pour une utilisation successive ou simultanée :

a) une chambre, par exemple une colonne, remplie d'un matériau graphite, de préférence du graphite ou du charbon activé, et

b) un jeu pour greffer un groupe protecteur comme défini ci-dessus sur une molécule.

Le graphite ou le matériau graphitisé a été défini ci-dessus, alors que le jeu pour le greffage du groupe protecteur comprend les divers réactifs connus des spécialistes comme pouvant greffer un groupe protecteur sur une molécule à protéger.

Cet appareil est avantageusement complété par (c) un système de collection de fraction équipé d'un détecteur d'ultraviolets (UV), spectroscopique visible ou spectroscopique fluorescent. Par exemple le groupe Tbfmoc peut agir comme un label fluorescent ; longueur d'onde d'excitation : 383 nm ; longueur d'onde d'émission : 424 nm. Ceci est particulièrement avantageux en immunologie, où la détection des composés présents dans les concentrations très faibles requiert des techniques à haute sensibilité.

Ainsi, il a été possible de développer un nouveau procédé pour la synthèse et/ou
la séparation des molécules, en particulier les fragments de peptides, qui comprend les étapes suivantes :

- protection d'au moins un groupe dans au moins un composé dans un mélange de composés à séparer avec un groupe comme défini ci-dessus, et
- passage du mélange de composés par une chambre remplie d'un matériau graphite.

La présente invention prévoit également l'utilisation d'un groupe comme défini ci-dessus pour protéger au moins un groupe fonctionnel d'une molécule.

La présente invention est maintenant plus amplement illustrée dans les Exemples suivants:

Les dérivés d'acides aminés ont été achetés soit chez Fluka, soit chez Aldrich ou Sigma. Les points de fusion ont été pris dans des capillaires ouverts sur un appareil de point de fusion Büchi 510 chauffé électriquement, ou sur des préparations de microscope en étape à chaud Reichert 7905 chauffée électriquement et ne sont pas corrigés. La chromatographie en fine couche (t.l.c.) a été effectuée en utilisant des feuilles de plastique revêtues de gel de silicium 60 GF-254 (Merck 5735) dans les systèmes suivants :

(A) éthyl acétate/pétrole (b.p. 40-60°C) (1 : 4)
(B) méthanol/chloroforme (1:9)
(C) toluène
(D) chloroforme
(E) éthyle acétate
(F) benzène
(G) dichlorométhane
(H) méthanol/chloroforme/acide acétique (1:9:0,5)
(I) n-butanol/acide acétique/eau (3:1:1)

La visualisation des composés a été réalisée par une combinaison appropriée des méthodes suivantes : vapeur diode, absorption d'ultraviolets à 254 nm ou 352 nm, aspersions de bleu de permanganate de potassium neutre et de bromophénol, réactif de Mary (diphényl carbinol (diméthylaminé) 4,4'-bis) pour les fonctions acides et ninhydrine pour les composés avec groupes aminés libres. Les rotations optiques ont été mesurées sur un polarimètre AA 1000 en utilisant une cellule de 1 dm dans les solvants cités dans le texte. Une chromatographie liquide haute performance (HPLC) a été effectuée en utilisant soit un système Waters, c'est-à-dire des pompes 2 x 600, un injecteur U6K, un contrôleur de gradiant automatique 680, un détecteur d'ultraviolet modèle 441; ou un système Applied Biossystems (Biosystèmes Appliqués), c'est-à-dire un système d'alimentation en solvant 2 x 1406 A, un injecteur/mixeur 1480 A et un contrôleur/détecteur 1783 A. Les séparations analytiques ont été exécutées sur un Whatman-Partisil-5 ODS-3 (4,6 mmDI x 250 mm) et sur un aquapore DE-300 ABI (RP18), taille de pore 300 A, colonne (4,6 mm DI x 220 mm) de silicium sphérique 7 µm dans les conditions indiquées dans le texte. la chromatographie éclair a été réalisée en utilisant du gel de silicium 60 (230-240 prise (Fluka) ; 60-100 g de silicium par gramme de matériau brut ; longueur active 15-20 cm). Les spectres infrarouges ont été enregistrés sur un spectrophotomètre Perkin Elmer 781 dans le solvant indiqué ou par la technique du disque KBr, en utilisant le polystyrène comme norme (1603 cm-1). Les spectres ultraviolets ont été enregistrés sur un spectrophotomètre à luminescence Perkin Elmer LS-5. Les spectres de masse ont été mesurés sur une machine Kratos MS 50 TC. Les spectres de résonnance magnétique nucléaire des protons (NMR) ont été enregistrés soit sur machines Brüker WP 80 (80 MHz), WP 200 (200 MHz) ou WH 360 (360 MHz) dans le solvant indiqué en utilisant du tétraméthylsilane comme norme ($\delta$ = 0,00) Les spectres au Carbone-13 NMR ont été enregistrés sur une machine Brüker WP 200 (50 MHz) ou WH 360 (90 MHz) dans le solvant indiqué, en utilisant le tétraméthylsilane comme norme. Les analyses élémentales ont été effectuées sur un analyseur élémental Carlo Erba modèle 1106. La détermination de la structure radiographique à cristal unique a été réalisée sur un diffractomètre à quatre cycles Stoë

Stadi-4 graphite-monochromé (radiation CU-Kδ; lambda = 1.54184 A). Tous les solvants ont été distillés avant utilisation et les suivants ont été séchés en utilisant les réactifs indiqués entre parenthèses quand besoin était: benzène (fil de sodium), chloroforme (pentoxyde de phosphore), dichlorométhane (hydrure de calcium), diéthyl éther (fil de sodium), dioxane (aluminés neutres et fil de sodium), méthanol (magnésium-iode), toluène (fil de sodium). Le pétrole (p.i. 40-60°C) se rapporte à la fraction qui entre en ébullition entre 40° C et 60° C.

EXEMPLE 1

Synthèse de 13-hydroxyméthyldibenzo(a,c)fluorène

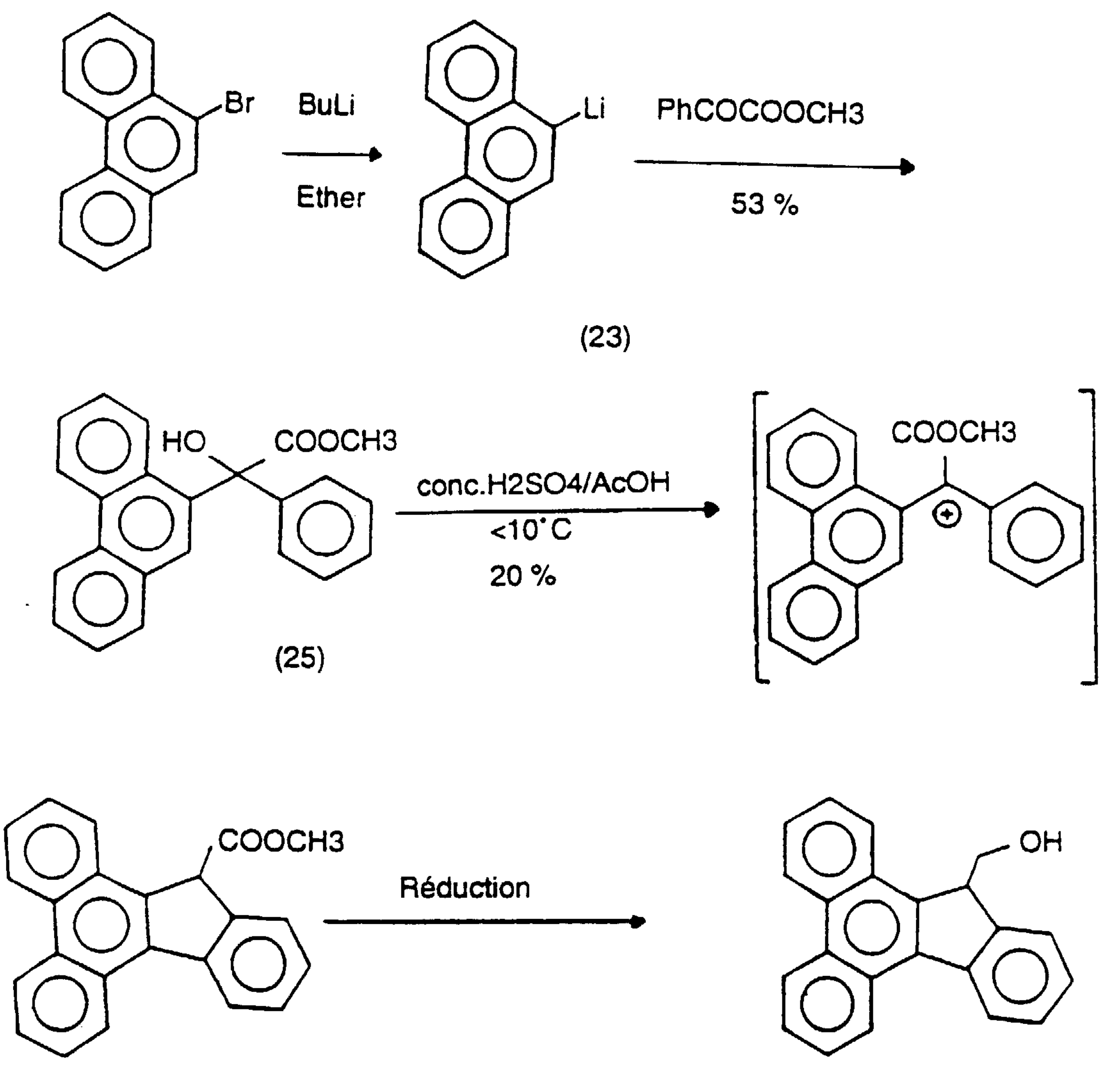


(23) (25) (26) (21)

Méthyl (phénanthrén-9-yl, phényl)hydroxaxétale (25)

La synthèse du méthyl (phénanthrén-9-yl, phényl) 5 hydroxyacétate (25) a été effectuée dans l'éther par réaction du lithium 9-phénanthrényl (23) avec méthyl benzoyl-formiate.

Une solution de n-butyllithium dans de l'hexane (8 ml, 11 mmol ; 1.1 équiv; titré 1.38M) a été ajoutée goutte à goutte pendant 10 mn à une solution à froid (0°C) de 9-bromophénanthrène (2,57 g, 10 mmol) dans de l'éther sec (10 ml) sous azote. Le mélange de réaction a été remué pendant une heure à température ambiante. On a laissé

reposer la suspension en résultant puis on a retiré le sumageant à la seringue. Le résidu a été resuspendu dans de l'éther (5 ml). Cette solution a été ajoutée, sous azote, à une solution à froid (0°C) de méthyl benzoylformiate (1,64 g, 1,4 ml, 10 mmol) dans 50 ml de diéthyléther. Le mélange résultant a été chauffé sous reflux pendant 2 heures et est resté à remuer pendant une nuit. Après addition de HCL (50 ml; pH=1) dilué (10 % V/V), la couche organique a été séparée, combinée avec des lavages d'éther (2 x 50 ml) de la couche aqueuse, lavée à l'eau (2 x 50 ml) et séché avec MgSO4. Le solvant a été retiré sous vide pour obtenir une huile jaune. Après purification par chromatographie éclair (éluant : (A)), une huile jaune a été obtenue qui a été filtrée et séchée dans un dessiccateur sous vide pour obtenir le composé requis (1,75 g, 51 %) ; p.f. 143-145°C (Trouvé : C, 79,7 ; H, 5,24 C23H1803 nécessite : C, 80,7 ; H, 5,26 %) ; t.l.c. Rf(A) 0,32 ; Rf(B) 0,76 ; pmax CH2CL2 3690 (OH libre), 3510 (OH lié H), 3025 (CH extension, aryl), 2960, 2880, 2860 (CH extension, alcoyl), 1730 (CO, ester), 1600, 1500 (anneaux aromatiques), 1225, 1185, 1170, 1110, 1100, 1070 (CO extension) cm-1; lambda max 296 (9832), 284 (9200), 276 (11981), 254 (48450), 248 (37620) nm ; δH (CDCl3, 200 MHz) 8,72 (1H, d, 3J = 8 Hz, aromatique), 8,16 (1H, d, 3J = 8 Hz, aromatique), 7,75-7,25 (11H, m, aromatique), 4,32 (1H, s, OH), 3,85 (3H, s, CH3) ; δC (CDCl3, 50 MHz) 175,7 (CO, ester), 141,1, 135,6, 131,3, 130,6, 130,3, 129,8 (C quatemaires aromatiques), 129,1, 122,9, 122,2 (CH aromatiques), 82,1 (C1, quatemaire), 53,4 (CH3); m/z (El) 342, 283, 105.

13-Carboxyméthyldibenzo(a,c)fluorène (26)

Le traitement de (25) dans des conditions acides (conc. H2S04, CH3COOH à 10°C) a mené à la formation de (26) en mauvais rendement.

De façon surprenante, le traitement de (25) avec PPA à 110°C a mené à la formation de l'ester souhaité (26) avec rendement de 42 %.

L'acide polyphosphorique (60 g) a été chauffé à 110°C et remué avec une palette mécanique. A cela a été ajouté du méthyl(phénanthrén-9-yl, phényl)hydroxyacétate

(5 g, 14,6 mmol). Le mélange a été remué pendant 1 heure à 110°C. La réaction a ensuite été refroidie à la température ambiante, diluée avec de l'eau (150 ml) et extraite avec de l'éthyl acétate (4 x 250 ml). Les couches organiques ont été combinées, lavées avec NaHCO3, (10% v/v ; 200 ml), de l'eau (100 ml) et séché sur MgSO4. Le solvant a été retiré in vacuo pour donner un solide jaune. Ce solide brut a été solidifié par chromatographie éclair en utilisant du toluène comme éluant. Après la chromatographie, le composé requis (2,82 g, 60 %) a pu être isolé. Ce solide a été recristallisé à partir de CH2Cl2/pétrole (b.p. 40-60°C) pour permettre un solide blanc, (1,96 g, 42%); mp. 190-191°C ; (Trouvé: C, 85,2 ; H, 4,93 C23H16O2 nécessite : C. 85,2 ; H, 4,94%) ; t.l.c. Rf(C) 0,50 ; Rf(D) 0,56 ; µmax (CH2Cl2), 3040, 3020 (CH extension, aryl), 2970 (CH extension, alcoyl), 1730 (CO, ester), 1610, 1600, 1580 (anneaux aromatiques) cm-1; lambda max 364 (540), 346 (16200), 322 (18360), 268 (64800) nm ; δH (CDCl3, 200 MHz), 8,84-8,67 (3H, m, aromatique), 8,36 (1H, d, 3J = 7,8 Hz, aromatique), 7,98-7,90 (1H, m, aromatique), 7,80-7,35 (7H, m, aromatique), 5,16 (1H, s, CH), 3,63 (3H, s, CH3) ; δ C(CDCl3, 50 MHz), 172,0 (CO, ester), 142,7, 141,9, 137,4, 135,6, 131,1, 130,0, 128,7, 128,4 (C aromatiques quatemaires), 128,1, 127,0, 126,7, 126,3, 126,2, 124,3, 124,1, 123,8, 123,3, 123,2, 122,9 (CH aromatiques), 53,4 (CH3), 52,4 (CH) ; m/z (El) 324, 265, 262, 132.

13-Hydrométhyldibenzo(a,c)fluorène (21)

La réduction de l'ester (26) a été réalisée en rendement de 48% en utilisant trois équivalents de l'hydrure de diisobutylaluminium (DIBAL-H) dans du dichlorométhane à -50° C.

Le hydrure de diisobutylaluminium(4,6 ml, 4,62 mmol; 1 M dans CH2Cl2) a été ajouté à -65°C à une solution de 1 3-carboxyméthyldibenzo(a,c)fluorène (0,5 g,

1,54 mmol) dans du dichlorométhane (10 ml). Le mélange de réaction a été remué pendant 3 heures et la température maintenue entre -50°C et -40°C. Un précipité blanc a été obtenu. Le mélange de réaction a été traité avec un mélange d'acide acétique et d'eau (1:1; 30 ml) et extrait avec du dichlorométhane (3 x 60 ml). La couche organique a été lavée avec du bicarbonate saturé (50 ml), de l'eau (30 ml) et séché sur MgSO4. ce solide brut a été purifié par chromatographie éclair en utilisant du chloroforme comme éluant. Le composé titre a été obtenu comme un solide blanc qui a été lavé avec du pétrole (0,217 g, 48%) ; m.p. 167-168°C ; (Trouvé : C, 89,0 ; H, 5,39 ; C22H160 nécessite : C, 89,2 ; H, 5,41%) ; t.l.c. Rf(D) 0,26; Rf(E) 0,64 ; µmax (CH2Cl2) 3610 (OH libre), 3490 (OH Lié H), 3060 (CH extension), 2940, 2890 (CH extension, alcoyl), 1610, 1600, 1580 (anneaux aromatiques) cm-1; lambda max 366 (888), 338 (12728), 322 (14504), 266 (27232), 246 (31968) nm ; δH (CDCl3, 200 MHz), 8,88-8,68 (3H, m, aromatique), 8,38 (1H, d, 3J = 7,9 Hz, ammatique), 8,14-8,08 (1H, m, aromatique), 7,81-7,33 (7H, m, aromatique), 4,45 (2H, m, HaCH2 et Hc), 3,85 (1H, m, Hb, CH2), 1,64 (1H, s, OH), δC (CDCl3, 50 MHz), 146,7, 142,5, 139,4, 134,9, 130,9, 130,1, 128,7 (C aromatiques quatemaires), 127,5, 126,8, 126,1, 125,9, 125,8, 124,7, 124,2, 124,1, 123,8, 123,4, 122,9 (CH aromatiques), 65,7 (CH2), 50,0 (CH); m/z (El) 296, 265.

Cette voie foumit une synthèse de 13-hydroxyméthyldibenzo(a,c)fluorène (21) en quatre étapes avec un rendement total de 10%.

Les acétates à la fois de l'alcool (21) et du 9-fluorèneméthanol (préparations montrées ci-dessous) ont ensuite été préparés afin de comparer leur comportement sur une colonne HPLC remplie de PGC.

L'acétate de 9-Fluorènylméthyl (29) a donné un délai de rétention de 3,3 mn (élué avec CHCl3), alors que celui de 13-acétoxyméthyldibenzo(a,c)fluorène (30) dans les mêmes conditions n'a pas été complètement retenu sur la colonne, mais a été lentement élué.

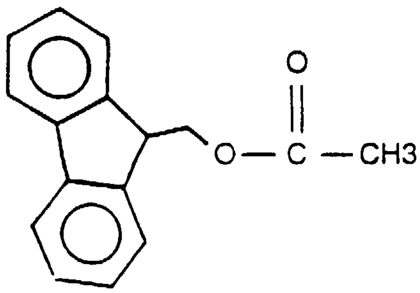

(29)

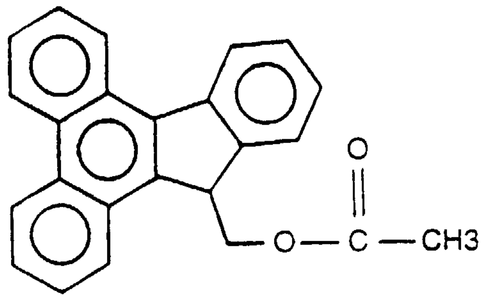

(30)

<u>Préparation de 13-Acétoxyméthyldibenzo(a,c)fluorène</u> (30)

13-Hydroxyméthyldibenzo(a,c)fluorène (215,2 mg, 0,727 mmol) a été dissous dans de l'anhydride acétique (5 ml). Une goutte d'acide sulfurique (2M) a été ajoutée à cette solution. Le mélange de réaction a été remué pendant 30 mn. Un précipité blanc a été obtenu, qui a été filtré et lavé avec de l'eau (150 ml). Le solide a enfin été séché à un poids constant sur P205 dans un dessiccateur sous vide pour donner 13-acétoxyméthyldibenzo(a,c)fluorène sous forme d'un solide blanc, (206,3 mg, 84%) ; m.p. 135-136°C ; (Trouvé: C, 84,9 ; H, 5,33 C24H18O2 nécessite : C, 85,2 ; H, 5,33%; t.l.c. Rf(D) 0,57 ; Rf(E) 0,68 ; pmax (CH2C12) 3030 (CH extension, aryl) 2980, 2920 (CH extension, alcoyl) 1735 (CO, ester), 1610, 1600, 1570 (anneaux aromatiques) cm-1; lambda max 366 (1184), 338 (25160), 322 (27528), 265 (52688), 246 (53020) nm ; δH (CDCl3, 200 MHz), 8,86-8,69 (3H, m, aromatique), 9,38 (1H, d, 3J = 7,9 Hz, aromatique), 8-30-8,25 (1H, m, aromatique), 7,78-7,35 (7H, m, aromatique), 5,16 (1H, dxd, 3Ja,c = 4,1 Hz, 2Ja,b = 10,8 Hz, Ha, CH2), 4,56 (1H, dxd, 3Jc,a = 4,1 Hz, 3Jc,b = 9,3 Hz, Hc), 3,76 (1H, dxd, 2Jb,a = 10,8 Hz, 3Jb,c = 9,3 Hz, Hb, CH2), 2,17 (3H, s, CH3) ; δC (CDCl3, 50 MHz) 171,0 (CO, ester), 146,7, 142,1, 138,7, 134,8, 131,1, 130,1, 128,7, 128,6 (C aromatiques quatemaires), 127,6, 127,0, 126,8, 126,2, 126,1, 125,7, 124,9, 124,8, 124,3, 123,4, 123,3, 122,9 (CH aromatiques), 67,3 (CH2), 46,5 (CH), 20,9 (CH3) ; m/z (El) 338, 277, 265, 139, 43.
HPLC : colonne (ODS3-PL5-393, solvants : A(H20), B(CH3CN), conditions : B(50%) 2 mn.

30 mn.
B (50%) --------> B (95%)

lambda = 254 nm, taux de débit : 1 ml/mn, injection : 5 µl, c = 1,42 mg/ml, AUFS = 2, délai de rétention : 21,6 mn.

<u>Préparation de 9-Fluorénylméthylacétate</u> (29)

9-Fluorénylméthanol (0,215 g, 1,1 mmol) a été dissous dans de l'anhydride acétique (5 ml). Deux gouttes d'acide sulfurique (2M) ont été ajoutées à cette solution. Celle-ci a été remuée pendant 30 mn. La solution claire a été versée sur de l'eau froide (20 ml). Le précipité obtenu a été récupéré sur un filtre Büchner et séché à un poids constant sur P205 dans un dessiccateur sous vide pour donner 9-fluorénylméthylacétate sous forme d'un solide blanc, (0,175 g, 67%) ; m.p. 83-84°C ; (Trouvé : C, 80,5 ; H, 5,98 C16H14O2 nécessite : C, 80,7; H, 5,88%) ; t.l.c. Rf(D) 0,50 ; Rf (E) 0,68 ; pmax (CHCl2) 3030 (CH extension), 2960, 2910 (CH extension, alcoyl), 1735 (CO, ester), 1610 (anneaux aromatiques) cm-1; lambda max 300 (2476), 290 (2063), 268 (7634) nm; δH (CDCl3, 80 MHz), 7,83-7,29 (8H, m, aromatique), 4-46-4,28 (3H, m, CH, CH2), 2,14 (3H, s, CH3) ; δC (CDCl3, 50 MHz), 170,8 (CO, ester), 143, 6, 141,1, (C aromatiques quatemaires), 127,6, 126,9, 124,9, 119,9 (CH aromatiques), 66,2 (CH2), 46,5 (CH), 20,8 (CH3) ; m/z (El) 238, 178, 165, 149, 60, 43.

HPLC : colonne (ODS3-PL5-393, solvants A(H20), B(CH3CN) Conditions : B(50%) 2mn.

30 mn.

B (50%) ---------> B (95%)

lambda = 254 nm, taux de débit : 1 ml/mn,
injection : 5 µl, C = 1,46 mg/ml, AUFS = 2, délai de rétention : 8,4 mn.

EXEMPLE 2

Synthèse de 17-Hydroxyméthyltétrabenzo(a,c,g,i)fluorène (31)

Le tétrabenzofluorényl alcool (31) a été préparé comme suit :

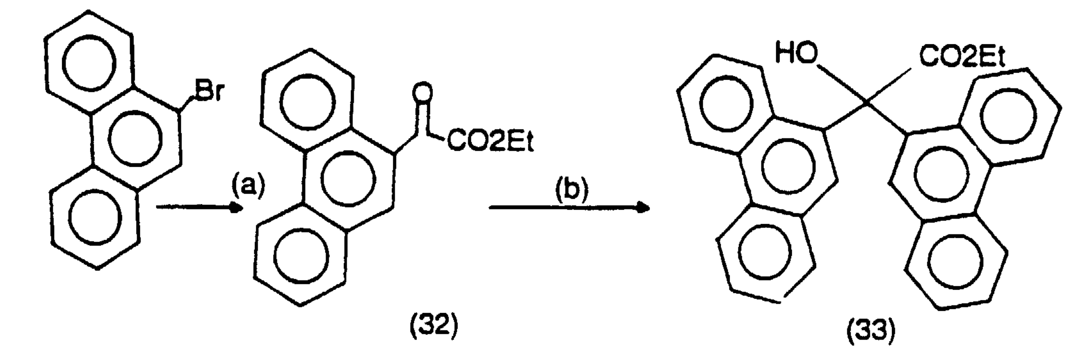


(32) (33)

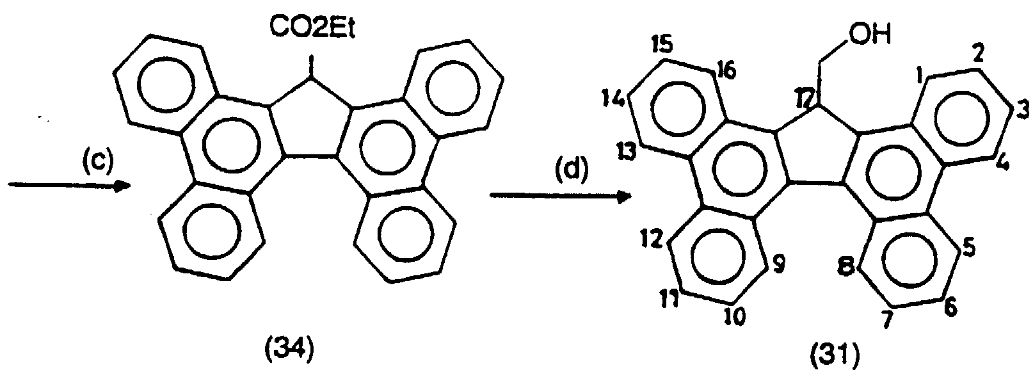


(34) (31)

(a) Buli, Et2O, d.r., 30 mn ; EtOOCCOOEt, Et2O, 0°C-5°C, 2h, 63%; (b) 9-Bromophénanthrène, Buli, Et2O, d.r., 30 mn ; (32), Et2O, 0°-5°C, 2h, 46%; (c) PPA, 140°C, 4h, 49%; (d) DIBAL-H (3 équiv.), CH2Cl2, 45°C, 1h, 73%.

Ethyl,2 oxo-2-(phénanthrén-9'-yl)acétate (32)

L'ester D-céto (32) a été synthétisé en ajoutant du 9-phénanthrényl lithium à une solution de diéthyl oxalate (20% excès) dans de l'éther à 0°C. Le mélange de réaction a été ensuite chauffé sous reflux pendant 2 heures. Après purification par chromatographie éclair, le produit a été isolé en rendement à 23%.
L'ester D-céto (32) peut aussi être avantageusement préparé dans l'éther, en ajoutant avec précaution du 9-phénanthrényl lithium à une solution de diéthyl oxalate (20% excès), entre 0°C et 5°C, et en remuant ensuite la réaction à la température ambiante. On a ajouté à 0°C sous azote une solution de n-butyllithium dans de l'hexane (16 ml, 22 mmol ; 1,1 équiv ; 1,38M titré) goutte à goutte à une solution remuée de 9-bromophénanthrène (5,14 g, 20 mmol) dans

de l'éther sec pendant 10 mn. la réaction a été remuée pendant 1 heure à température ambiante. On a laissé reposer la suspension en résultant et le sumageant a été retiré à la seringue. Le résidu a été suspendu de nouveau dans de l'éther (10 ml). Cette suspension a été ajoutée sous azote à une solution à froid (0°C) de diéthyl oxalate (3,4 ml, 25 mmol) dans de l'éther (100 ml). La température a été maintenue entre 0°C et 5°C pendant l'addition. Le mélange de réaction a ensuite été remué pendant 2h entre 0° et 5°C et finalement à température ambiante pendant 2h. Après addition d'HCl dilué (100 ml ; 10% v/v), la couche organique a été séparée, combinée avec des lavages d'éthyl acétate (3 x 100 ml) de la couche aqueuse, neutralisée avec une solution de NaHCO3 (100 ml ; 1M), lavée avec de l'eau ((50 ml) et séchée sur MgSO4. Le solvant a été retiré in vacuo pour donner une huile orange qui a été homogénéisée avec du pétrole (p.i. 40°C-60°C). Le composé titre a été obtenu sous la forme d'un solide jaune qui a été filtré et séché, (3,53 g, 63%); p.f. 67-68°C ; (Trouvé: C, 77,5 ; H, 5,02 C18H14O3 nécessite : C, 77,7 ; H, 5,04%) ; t.l.c. Rf(A) 0,56, Rf(D) 0,52 ; pmax (CH2Cl2) 3070 (CH extension, aromatique), 2990, 2940, 2910, 2880 (CH extension, alcoyl), 1735 (CO, ester), 1680 (CO, cétone), 1620, 1575 (anneaux aromatiques) cm-1; lambda max 328 (9822), 286 (7784), 252 (34842) nm ; δH (CDCl3, 200 MHz) 9,04 (1H, m, aromatique), 8,62 (2H, m, aromatique), 8,23 (1H, s, H10 aromatique), 7,92 (1H, d, 3J = 7,6 Hz, aromatique), 7,78-7,59 (4H, m, aromatique), 4,51 (2H, 9, 3J = 7,1 Hz, CH2), 1,48 (3H, t, 3J = 7,1 Hz, CH3), δC (CDCl3, 50 MHz) 188,5 (CO, cétone), 164,4 (CO, ester), 137,2 (CH aromatique), 132,8 (aromatique quatemaire), 130,5, 130,2 (CH aromatiques), 129,2, 128,1 (C aromatiques quatemaires), 128,0, 127,4, 127,1, 126,3, 122,7, 122,6 (CH aromatiques), 62,3 (CH2), 14,0 (CH3) ; m/z (EI) 278, 205, 177, 176.

Ethyl(bis-phénantrèn-9'-y)hydroxy acétate

L'alcool tertiaire (33) a été synthétisé en rendement approprié dans des conditions similaires en utilisant (32) comme composant céto ester.

On a ajouté une solution de n-butyllithium dans de l'hexane 127 (8 ml, 11 mmol; 1,1 équiv. ; 1,38 M titré) à une solution de 9-bromophénanthrène (2,57 g, 10 mmol) dans de l'éther sec (10 ml), à 0°C sous azote goutte à goutte pendant 10 mn. Le mélange de réaction a été remué pendant une heure à température ambiante. Cette solution a été ajoutée sous azote à une solution à froid (0°C) d'éthyl, 2-oxo-2(phénanthrèn-9'-yl)acétate (2,78 g, 10 mmol) dans de l'éther (50 ml). La température a été maintenue entre 0°C et 5°C pendant l'addition. Le mélange de réaction a finalement été remué à température ambiante pendant deux heures. Après addition de HCl dilué (50 ml; 10% v/v) la couche organique a été séparée, combinée avec des lavages d'éthyl acétate (3 x 250 ml) de la couche aqueuse, neutralisée avec une solution de NaHCO3 (1M; 200 ml), lavée avec de l'eau (200 ml) et finalement séchée sur MgSO4. Le solvant a été retiré in vacuo pour obtenir un résidu. Après purification par chromatography éclair utilisant du chloroforme/ pétrole (p.i. 40°C-60°C) (4 : 1), le produit attendu a été obtenu à partir des fractions contenant du matériau de Rf = 0,23. Après recristallisation du dichlorométhane/pétrole (p.i. 40°C-60°C), un solide blanc a été obtenu qui a été filtré et séché pour donner le composé titre (2,11 g, 46%); p.f. 188-189°C ; (Trouvé: C, 83,1; H, 5,23 C32H24O3 nécessite : C, 84,2 ; H, 5,26%) ; t.l.c. Rf (E) 0,71; Rf (D) 0,48 ; pmax (CH2Cl2) 3680 (OH libre), 3510 (OH lié H), 3060 (CH extension, aromatique), 2990, 2940 (CH extension, alcoyl), 1735 (CO, ester), 1600 (anneau aromatique) cm-1; lambda max 332 (629), 300 (25080), 288 (23560), 256 (123120) nm; δH (CDCl3, 200 MHz), 8,80-8,69 (4H, m, aromatique), 8,51 (1H, s, H10, aromatique), 8,47 (1H, s, H10, aromatique), 7,72-7,41 (12H, m, aromatique), 4,46 (1H, s, OH), 4,41 (2H, 9, 3J = 7,1 Hz, CH2), 1,17 (3H, t, 3J = 7,1 Hz, CH3), δC (CDCl3, 50 MHz) 1/5,7 (CO, ester), 135,0, 131,4, 130,6, 130,5, 130,1 (C aromatiques quatemaires), 129,2, 128,2, 127,3, 126,6, 126,2, 126,1, 122,9, 122,3 (CH aromatiques), 84,3 (C1, quaternaire), 62,9 (CH2), 13,8 (CH3); m/z (EI) 383, 206, 177, 176.

17-Carboxyéthyltétrapenzo(a,c,g,i)fluorène (34)

Le traitement de (33) avec de l'acide polyphosphorique à 140°C a mené à la formation d'ester cyclique (34) en rendement à 49% via 4-électrocyclisation du cation D-éthoxycarbonyl diaryl.

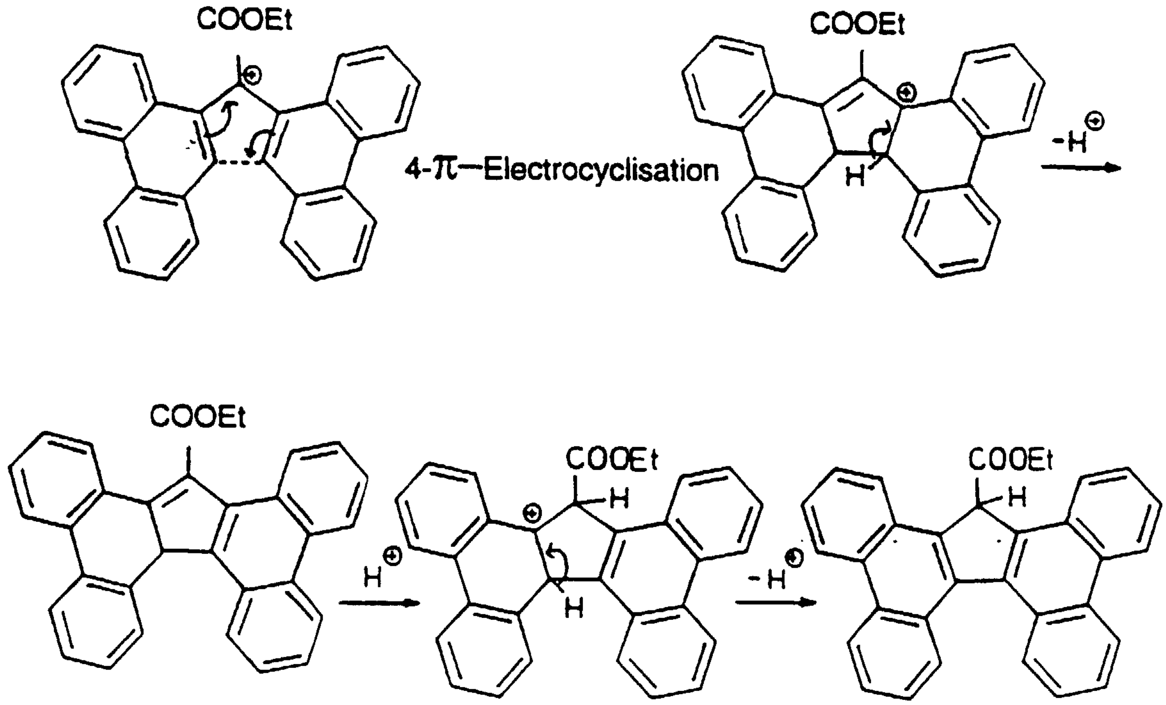


(34)

La purification de (34) est difficile du fait de la formation de produits latéraux. La recristallisation du matériau brut à la suite de la chromatographie éclair peut être réalisée. De l'acide polyphosphorique (20 g) a été placé dans un récipient à fond rond de 100 ml à trois goulots équipé d'une palette mécanique pour remuer. De l'éthyl (bis-phénanthrèn-9'-yl) hydroxy acétate (0,402 mg, 0,881 mmol) a ensuite été ajouté et la réaction a été remuée à 140°C pendant quatre heures puis à température ambiante jusqu'au lendemain. Le mélange de réaction a été dilué avec de l'eau (50 ml) et extrait avec de l'éthyl acétate (4 x 50 ml). La couche organique a été neutralisée avec $NaHCO_3$ (1M ; 2 x 30 ml), lavée avec de l'eau (30 ml).

Après la chromatographie éclair et la recristallisation de DCM, des cristaux de (31) ont été obtenus et se sont avérés appropriés à une analyse radiographique.De l'hydrure de diisobutylaluminium (12,3 ml, 12,3 mmol ; 3 équiv. ; solution de 1M dans $CH_2Cl_2$) a été ajouté goutte à goutte à -65°C à une solution de 17-carboxyéthyltétrabenzo(a,c, g,i)fluorène (1,795 g, 4,098 mmol) dans du dichlorométhane distillé à sec (25 ml). La température a été maintenue entre -65°C et -60°C pendant l'addition. Le mélange de raction a été remué pendant une heure à -65°C et à la température ambiante pendant encore une heure. Le mélange de réaction a été refroidi à -30°C. Une solution aqueuse d'acide acétique (50 ml ; 10% v/v) a ensuite été ajoutée goutte à goutte. Après séparation des deux couches, la phase aqueuse a été extraite avec du dichlorométhane (3 x 100 ml). Les phases organiques combinées ont été lavées avec de l'eau (70 ml) et neutralisées avec du $NaHCO_3$. Enfin, la phase organique a été séchée sur du $MgSO_4$ et évaporée pour donner une huile brute (1,7 g). Après purification par chromatographie éclair utilisant du benzène comme éluant, les fractions contenant du matériau de Rf = 0,14 ont été évaporées pour donner un solide jaune. Celui-ci a été recristallisé à partir de $CH_2Cl_2$/pétrole (p.i. 40°C-60°C) pour donner le composé titre sous forme d'un solide jaune (1,18g, 73%°) ; p.f. 202-203°C ; (Trouvé: C, 91,2; H, 4.96 $C_{30}H_{20}O$ nécessite : C, 90,9; H, 5,05%) ; t.l.c. Rf (F) 0,15, Rf (A) 0,18, Rf (B) 0,75 ; µmax ($CH_2Cl_2$) 3600 (OH libre), 3060 (CH extension, aromatique), 2940, 2900 (CH extension, alcoyl), 1610, 1500 (anneaux aromatiques), 1045 (CO extension) $cm^{-1}$; lambda max 380 (10692), 368 (11484), 302 (27324), 290 (21780), 254 (43560) nm ; δH ($CDCl_3$, 200 MHz), 8,80-8,63 (6H, m, aromatique), 8,27-8,22 (2H, m, aromatique), 7,73-7,56 (8H, m, aromatique), 5,05 (1H, t, 3J = 4,3 Hz, CH), 4,49 (2H, d, 3J = 4,3 Hz, $CH_2$), 1,31 (1H, s, OH) ; δC ($CDCl_3$, 50 MHz), 141,6, 137,3, 131,4, 130,4, 128,5, 127,9 (C aromatiques quatemaires), 127,4, 126,9, 126,1, 125,9, 125,0, 124,5, 123,4 (CH aromatiques), 66,5 ($CH_2$), 50,8 (CH) ; m/z (EI) 396 (M+), 366.

La structure des cristaux de l'alcool (31) indique qu'il y a deux molécules maintenues ensembles par une liaison d'hydrogène entre les deux groupe hydroxyl. Les Figures 1 et 2 montrent la structure des cristaux de l'alcool (31). Le groupe hydroxyméthyl exocyclique a deux conformations différentes, probablement parce que c'est nécessaire

à la formation du dimère lié par hydrogène. L'intéraction entre H8 et H9 (d = 2,03 A, rayon de Van der Waal pour l'hydrogène = 1,2 A) est responsable d'un degré de non-planarité dans la molécule. Cependant, la molécule est suffisamment planaire pour les besoins de l'invention. Cette molécule est chirale si elle adopte une orientation fixe, comme dans la structure des cristaux. Donc, il est possible que dans une solution à basse température, la molécule puisse également adopter une orientation fixe et qu'elle soit chirale. 1H R.M.N. à température ambiante dans CDCl3 a montré un triplet (δ = 5,05 ppm) ainsi qu'un doublet (δ = 4,5 ppm), correspondant à H(17) et les deux protons du groupe méthylène respectivement. Une explication probable est que les anneaux de phénanthrène peuvent osciller rapidement pendant la période d'acquisition et par conséquent les protons du groupe CH2 sont magnétiquement équivalents (comme le sont les paires correspondantes de protons aromatiques, par exemple H(2) et H(15).

Les expériences nucléaires d'Overhauser effectuées sur ce composé ont démontré les interactions étroites dans l'espace entre le groupe méthylène et les deux protons aromatiques H(1) ET H(16).

EXEMPLE 3

Synthèse de 17-acétoxyméthyltétrabenzo(a,c,g,i)fluorène (35)

17-acétoxyméthyltétrabenzo(a,c,g,i)fluorène (35) a été facilement préparé dans un rendement à 80% d'alcool (31) en utilisant un grand excès d'anhydride acétique et une quantité catalytique d'acide sulfurique.

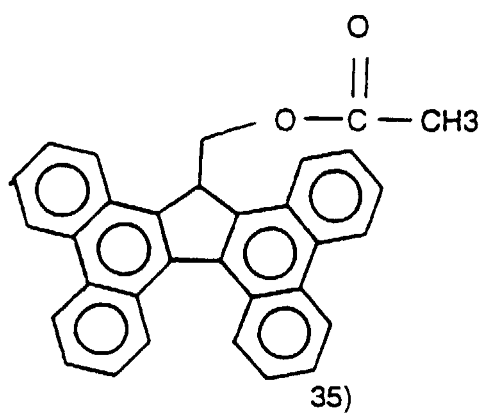

35)

Comme prévu, l'acétate (35) a été totalement retenu une fois passé par une colonne HPLC remplie de PGC (éluant CHCl3).

Du 17-Hydroxyméthyltétrabenzo(a,c,g,i)fluorène (0,207 g, 0,522 mmol) a été dissous dans de l'anhydride acétique (4 ml). 2 gouttes de H2SO4 (2M) ont été ajoutées à cette solution. Le mélange de réaction a été remué pendant 1 heure. Le composé titre a été obtenu sous la forme d'un solide jaune qui a été lavé avec de l'eau (80 ml) et séché à poids constant sur du P205 dans un dessiccateur sous vide (183,8 mg, 80%); p.m. 209-210°C, (Trouvé : C, 87,0 ; H, 5,08 C32H22O2 nécessite : C, 87,7 ; H, 5,02%) ; t.l.c. Rf (A) 0,32, Rf (F) 0,22 ; µmax (CH2Cl2) 3060 (CH extension, aromatique), 1740 (CO, ester), 1610, 1500 (anneaux aromatiques), 1230, 1045 (CO, extension) cm-1; lambda max 380 (17885), 368 (19345), 302 (45260), 290 (36865), 254 (72271) nm; δH (CDCl3, 80 MHz), 8,83-8,57 (6H, m, aromatique), 8,29-8,17 (2H, m, aromatique), 7,78-7,54 (8H, m, aromatique), 5,12 (1H, t, 3J = 5,5 Hz, CH), 4,60 (2H, d, 3J = 5,5 Hz, CH2), 1,84 (3H, s, CH3) ; δC (CDCl3, 50 MHz) 170,7 (CO, ester), 141,8, 137,0, 131,5, 130,4, 128,6, 128,0 (C aromatiques quatemaires), 127,4, 126,8, 126,1, 126,0, 125,0, 124,9, 123,5, 123,3 (CH aromatiques), 67,9 (CH2), 47,1 (CH), 20,7 (CH3) ; m/z (El) 438 (M+), 378, 364.

EXEMPLE 4

Conversion de 17-hydroxyméthyltétrabenzo (a,c,g,i) fluorène en chloroformiate

Le chloroformiate ((37) est synthétisé par la séquence de réactions suivante : traitement de l'alcool (31) avec de l'urée N, N'-bis-triméthylsilyl (2,5 équiv) pour donner l'éther triméthylsilyl (39) correspondant, suivi par la réaction de ce demier avec du phosgène. Après la recristallisation, le produit pur peut être obtenu en rendement de 21%.

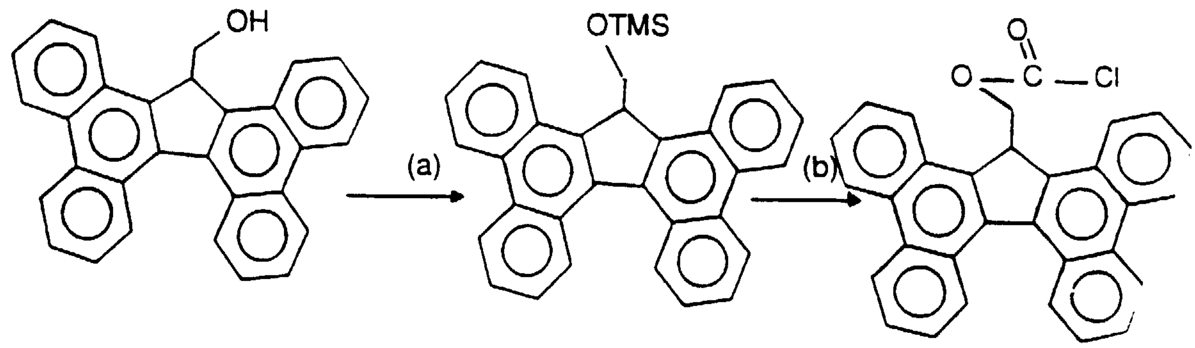


(31) (39) (37)

(a) Urée N,N'-bis-triméthylsilyl (2,5 équiv), CH2Cl2, reflux, 3h, 81%; (b) phosgène (7,5 équiv), CH2Cl2, reflux, 2h, 21%.

17-(Triméthylsilyl)oxyméthyltétrabenzo(a,c,g,i)fluorène (39)

Une solution de 17-hydroxyméthyltétrabenzo(a,c,g,i) fluorène (0,05 g, 0,126 mmolj et d'urée N,N'-bis-triméthylsilyl (32,3 mg, 0,158 mmol ; 2,6 équiv.) dans du dichlorométhane (2 ml) a été chauffée sous reflux pendant trois heures. L'urée précipitée a été filtrée et lavée avec du dichlorométhane (2 x 1 ml). Le solvant a été retiré in vacuo pour donner un résidu. Une purification par chromatographie éclair humide (benzène/pétrole (p.i. 40-60°C) (75:25)) a donné le composé titre (47,8 mg, 81%) ; p.f. 130-131°C ; (Trouvé: C, 84,2 ; H, 6,06 C33H28O Si nécessite : C, 84,6 ; H, 5,98%) ; t.l.c. Rf (F) 0,58, Rf (A) 0,51, Rf (G) 0,76 ; δH (CDCl3, 80 MHz) 8,86-8,62 (6H, m, aromatique), 8,49-8,37 (2H, m, aromatique), 7,79-7,51 (8H, m, aromatique), 5,11 (1H, t, 3J = 5,3 Hz, CH), 4,13 (2H, d, 3J = 5,3 Hz, CH2), 0,4 (9H, s, 3xCH3) ; δC (CDCl3, 67 MHz) 143,4, 136,5, 131,4, 130,3, 129,0, 128,1 (C aromatiques quatemaires), 127,4, 126,5,125,9, 125,8, 125,6, 124,9, 123,5, 123,0 (CH aromatiques), 67,2 (CH2), 51,4 (CH), -1,0 (3xCH3); m/z (El) 468 (M+), 378, 364.

17-Tétrabenzo(a,c,g,i)fluorénylméthyl chloroformiate (37)

Du phosgène (1,5 ml, 2,9 mmol; 7,5 équiv; 1,93 M dans le toluène) a été ajouté à une solution de 1 7-(triméthylsilyl)oxyméthyltétrabenzo(a,c,g,i)fluorène (0,177 g, 0,38 mmol) dans du dichlorométhane (5 ml). Le mélange de réaction a été chauffé sous reflux pendant 2 heures puis remué à la température ambiante pendant 48 heures, sous azote. Le solvant a été retiré in vacuo pour donner le composé titre sous forme d'un solide jaune qui a été recristallisé à partir de dichlorométhane/n-hexane (36,9 mg, 21%) ; p.f. 188-189°C ; (Trouvé: C, 80,9 ; H, 4,29 ; N, 0,45 C31H19O2Cl nécessite : C, 81,1; H, 4,14%) ; µmax (CH2Cl2) 3060 (CH extension, aromatique), 1775 (CO, chloroformiate), 1610, 1500 (anneaux aromatiques), 1160, 1140 (CO, extension) cm-1; δH (CDCl3, 200 MHz) 8,79-8,60 (6H, m, aromatique), 8,20-8,15 (2H, m, aromatique), 7,76-7,57 (8H, m, aromatique), 5,17 (1H, t, 3J = 5,7 Hz, CH), 4,77 (2H, d, 3J = 5,7 Hz, CH2) ; δC (CDCl3, 50 MHz) 150,5 (CO, chloroformiate), 140,3, 137,2, 131,6, 130,5, 128,2, 127,7 (C aromatiques quatemaires), 127,5, 127,0, 126,3, 126,2, 125,1, 124,5, 123,5, 123,4 (CH aromatiques), 74,6 (CH2), 46,5 (CH).

EXEMPLE 5

Préparation de N-17-tétrabenzo(a,c,g,i)fluorénylméthoxycarbonylglycine (Tbfmoc Gly OH)

Tbfmoc Gly OH a été synthétisé en trois étapes de à partir de l'acool (31) via le carbonate p-nitrophényl (41).

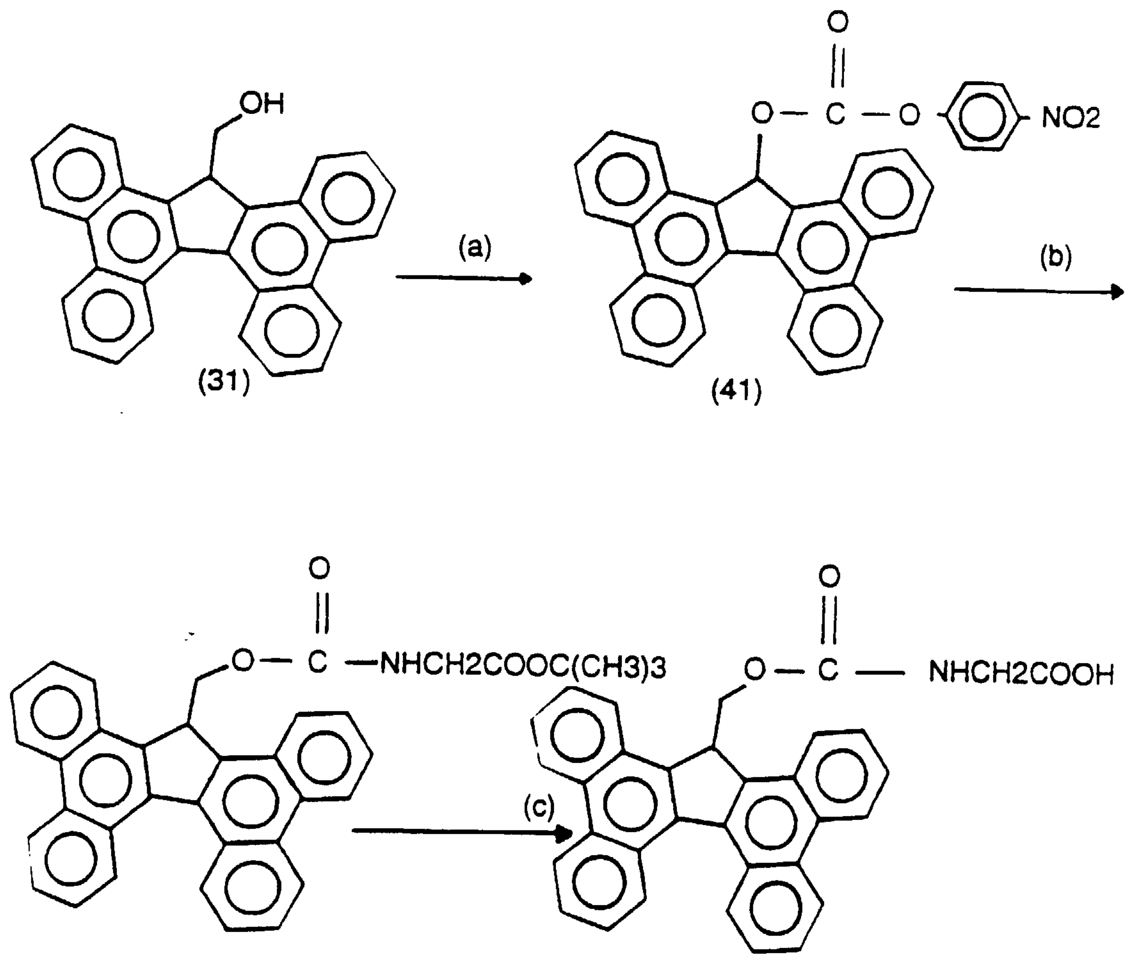


(31) (41)

(42) Tbfmoc Gly OH (36)

(a) <u>para</u>-Nitrophényl chloroformiate (2 équiv), N,N'-diméthylaniline (1 équiv), CH2Cl2, d.r., 72h, 80%; (b) CH3COOH. NH2CH2COOC(Me)3, N,N'-diméthylaniline (2 équiv), CH2Cl2, d.r., 24h, 79%; (c) acide <u>para</u>-toluènesulfonique (0,3 équiv), CH2CCl2, reflux, 5h, 90%.

La réaction du chloroformiate <u>para</u>-nitrophényl (2 équiv) avec de l'alcool (31) et N,N'-diméthylaniline a donné le carbone mélangé (41) en rendement de 80%. Ce matériau a à son tour réagi avec le sel d'acétate de glycine <u>tert</u>-butyl ester en présence de deux équivalents de N,N'-diméthylaniline pour donner la glycine tert-butyl ester protégée (42) (79%). Une simple hydrolise de cette dernière en utilisant de l'acide p-toluènesulfonique dans le DCM en reflux a donné l'acide souhaité (36) en rendement de 90%. Ceci est un synthèse facile de Tbfmoc Gly OH utilisant des conditions légèrement basiques dans un rendement total à 57% basé sur l'alcool (31).

<u>17-Tétrabenzo(a,c,g,i)fluorénylméthyl-p-nitrophényl carbonate</u> (41)

De la N,N'-diméthylaniline (16 µl, 0,126 mmol) a été ajoutée à une solution de 17-hydroxyméthyl tétrabenzo (a,c,g,i)fluorène et de chloroformiate p-nitrophényl (35,5 mg, 0,175 mmol ; 1,4 équiv.) dans du dichlorométhane (2 ml). La réaction a été remuée à la température ambiante sous azote pendant 24h. La réaction a été complétée en ajoutant plus de chloroformiate p-nitrophényl (45 mg, 0,233 mmol ; 1,7 équiv.) et en la remuant à la température ambiante pendant 48 heures de plus. Après purification par chromatographie éclair en utilisant du toluène comme éluant, les fractions contenant le matériau de Rf=0,29 ont été évaporées pour donner une huile jaune. L'homogénéisation dans du pétrole (p.. 40-60°C) a donné le <u>composé titre</u> sous la forme d'un solide jaune (56,5 mg, 80%); p.f. 139-140°C ; (Trouvé : C, 77,7 ; H, 3,94 ; N, 2,29 C37H23NO5 nécessite : C, 79,1; H, 4,10 ; N, 2,49%); t.l.c. Rf (C) 0,29, Rf (A) 0,16 ; pmax (CH2C12) 3060 (CH extension, aromatique), 2960, 2930, 2880 (CH extension, alcoyl), 1770 (CO, carbonate), 1620, 1600, 1495 (anneaux aromatiques), 1530, 1350 (nitro-NO2 conjugués), 1215 (CO extension), 860 (CH courbure, p-disubstitué) cm-1; lambda max 366 (20035) 301 (52092), 289 (48085), 260 (85751), 252 (88957) nm; δH (CDCl3, 200 MHz) 8,79-8,76 (4H, m, aromatique), 8,61-8,57 (2H, m, aromatique), 8,33-8,16 (2H, m, aromatique), 7,91 (2H, d,

JAB = 8,9 Hz, p-nitrophényl), 7,76-7,56 (8H, m, aromatique), 6,64 (2H, d, JAB = 8,9 Hz, p-nitrophényl), 5,14 (1H, t, 3J = 4,7 Hz, CH), 4,96 (2H, d, 3J = 4,7 Hz, CH2); δC (CDCl3, 50 MHz) 154,9 (CO, carbonate), 151,6 (C1' aromatique quatemaire), 144,9 (C-4'aromatique quaternaire), 140,3, 137,4, 131,5, 130,4, 128,3, 127,7 (c aromatiques quatemaires), 127,4, 127,0, 126,2, 125,1, 124,7, 124,1, 123,5, 121,2 (CH aromatiques), 125,3 (CH2', 6' aromatique), 121,4 (CH3'5' aromatique), 71,2 (CH2), 46,8 (CH) ; m/z (El) 378, 139, 44 ; (FAB) 561 (M+), 379. HRMS 561.15759, C37H23NO5 nécessite : 561.15761 ( ) <1 ppm.

N-17-Tétrabenzo(a,c,g,i)fluorénylméthoxycarbonylglycine tert-butyl ester (42)

De l'aniline N,N'-diméthyl (18 µl, 0,14 mmol ; 2 équiv) a été ajoutée à une solution de 17-tétrabenzo(a,c,g,i) fluorénylméthyl-p-nitrophényl carbonate (39,2 mg, 0,07 mmol) et de sel d'acétate glycine tert-butyl ester (14,7 mg, 0,077 mmol ; 1,1 équiv.). Le mélange de réaction a été remué à la température ambiante, sous azote, pendant 72 heures. Après addition d'eau (10 ml) et acidification avec KHSO4 (2M ; pH=1), le mélange de réaction a été extrait avec du dichlorométhane (3 x 15 ml), lavé avec de l'eau (3 x 20 ml) et séché sur du MgSO4. Le solvant a été retiré in vacuo pour donner une huile orange qui a été homogénéisée dans de l'éther. Un solide jaune a été obtenu qui a été filtré, lavé avec du pétrole (p.i. 40-60°C) et séché pour donner le composé titre (30,6 mg, 79%) ; p.f. 170-171°C ; (Trouvé : C, 79,7 ; H, 5,51; N, 2,33 C32H3NO4 nécessite : C, 80,3 ; H, 5,60 ; N, 2,53%) ; t.l.c. Rf (H) 0,79, Rf (I) 0,95 ; µmax (CH2Cl2) 3440 (NH amidé secondaire), 3060 (CH extension, aromatique), 2940 (CH extension, alcoyl), 1725 (CO, uréthane, ester et amidé I), 1660, 1500 (anneaux aromatiques), 1520 (amidé II), 1340 (lien OH) cm-1, 1220, 1160, 1110 (CO extension), 865, 850 (lien CH hors plan); lambda max 384 (16323), 367 (17656), 302 (42641), 290 (34313), 262 (62296) nm ; δH (CDCl3, 80 MHz) 8,85-8,60 (6H, m, aromatique), 8,39-8,28 (2H, m, aromatique), 7,81-7,56 (8H, m, aromatique), 5,27 (1H, t, 3J = 6 Hz, CH), 4,98 (1H, s, large, NH), 4,61 (2H, d, 3J = 6 Hz, CH2), 3,74 (2H, d, 3J = 6 Hz, CH2 glycine), 1,44 (9H, s, CH3x3) ; δC (CDCl3, 50 MHz), 168,7 (CO, ester), 156,1 (CO, uréthane), 141,9, 136,7, 131,5, 130,3, 128,7, 127,9 (C aromatiques quatemaires),127,4,126,8,126,0, 125,8, 125,3, 124,9, 123,5, 123,1 (CH aromatiques), 82,0 (C quatemaire, CMe3), 69,0 (CH2), 47,5 (CH), 43,2 (CH2, glycine), 27,9 (CH3x3) ; m/z (FAB) 553,379. HRMS 553.22527, C32H31NO4 nécessite 553.225529 ( ) < 1 ppm.

N-17-Tétrabenzo(a,c,g,i)fluorénylméthozycarbonyl glycine, Tbfmoc GLYOH (36)

Une solution de N-17-tétrabenzo(a,c,g,i)fluorényl méthoxycarbonyl glycine tert-butyl ester (0,392 g, 0,708 mmol) et d'acide p-toluènesulfonique (39,2 mg, 0,206 mmol) dans du dichlorométhane (15 ml) a été chauffé sous reflux pendant 4,5 heures, au cours desquelles un précipité s'est formé. Ce précipité a été filtré, lavé avec du dichlorométhane (2 x 15 ml) et séché pour donner le composé titre (319 mg, 90%) ; p.f. 240-241°C ; (Trouvé: C, 77,7 ; H, 4,5 ; N, 2,0 C33H23NO4 nécessite : C, 79,7 ; H, 4,63 N, 2,82%) : tlc Rf (H) 0.56, Rf (I) 0.80 (νmax) (disque KBr) 3320 (NH extension). 3060 (CH extension, aromatique), 1735 (CO, acide), 1710 (CO, uréthane), 1680 (amidé I), 1540 (amidé II), 1610, 1500 (anneaux aromatiques), 1435 (CH déformations, alcoyl), 1310 (OH courbure), 1260, 1240, 1175, 1160, 1040 (CO extension), 1000, 745, 720 (courbure CH hors plan) cm-1; lambda max 382 (21744), 368 (23297), 303 (55913), 291 (45041), 264 (82005), 255 (88839) nm; δH (d-dioxane, 200 MHz), 9,05-8,97 (4H, 2xd, aromatique), 8,83 (2H, d, 3J = 7,8 Hz, aromatique), 8,64 (2H, d, 3J = 7,8 Hz, aromatique), 7,95-7,74 (8H, m, aromatique), 6,53 (1H, t, 3J = 5,8 Hz, NH), 5,61 (1H, t, 3J = 5,8 Hz, CH2 glycine) ; δC (d-dioxane, 50 MHz) 170,6 (CO, acide), 156,2 (CO, uréthane), 142,5, 136,2, 131,3, 130,0, 128,7, 127,7 (C aromatiques quatemaires), 127,0, 126,5, 125,6, 125,4, 124,5, 123,3, 122,8 (CH aromatiques), 68,3 (CH2), 47,6 (CH), 41,5 (CH2, glycine) ; m/z (FAB) 497, 397. HRMS 498.17053, C33H24N04 nécessite 498.17052 ( ) < 1 ppm.

HPLC : colonne (RP 18), solvants : A (H2O/TFA(0,1%)), B(CH3CN/TFA(0,1%); conditions : B (75%), A (25%) ; lambda=229 nm, AUFS=2 ou lambda=368 nm, AUFS=1; taux de débit : 1 ml/mn ; injection ; 25 µl, C=1,3 mg/ml (dioxane/eau (1:1)), délai de rétention : 5,4 mn.

EXEMPLE 6

Synthèse en phase solide

Tous les dérivés des acides aminés protégés ont été achetés chez Novabiochem et ont une stéréochimie-L. La synthèse des peptides en phase solide a été effectuée sur un synthétiseur à peptides 430 A de Applied Biosystems (Biosystèmes Appliqués). Le DMF utilisé a été foumie par Rathbum Chemicals Ltd. (grade de synthèse des peptides). Le premier résidu de chaque séquence (c'est-à-dire le résidu terminal-C) a été couplé à la résine d'alcool p-alkoxbenzyl à l'extérieur du synthétiseur. L'étendu de l'accouplement a été déterminée par la déprotection d'un échantillon-réduit de la résine chargée et par un contrôle quantitatif de l'oléfine produite par UV à 300 nm dans le cas des peptides-Fmoc et à 364 nm dans le cas des peptide Tbfmoc. Chaque résidu a été couplé deux fois (2 équiv.), d'abord par la méthode

d'anhydride symétrique et ensuite par la méthode d'ester actif HOBt ("double accouplement") (sauf Arg, Asn, Gln couplés en double via ester HOBt et Gly couplé une fois via anhydride symétrique (4 équiv.). Des anhydrides symétriques préformés ont été préparés à partir d'acides Fmoc-aminés (1 mmol) et DICI (0,5 mmol) avec un délai d'activation de 15 minutes, et des esters HOBt à partir d'acides Fmoc-aminés (0,5 mmol), DICI (0,5 mmol) et HOBt (0,5 mmol) avec un délai d'activation de 30 minutes. Les réactions d'accouplement ont été effectuées sur une période de 30-60 minutes. L'amorçage des fonctions aminées non réagies a été exécuté pendant 6 minutes en utilisant de l'anhydride acétique et de la pyridine dans la DMF. La déprotection de résine-peptide-Fmoc a été réalisée sur une période de 12 minutes (5+3+3+1 minutes) en utilisant une solution de piperidine à 20% dans la DMF. La résine a été complètement lavée avec de la DMF à la fin de chaque cycle. En tant qu'élément de surveillance brut des accouplements, la solution du produit des étapes de déprotection a été alimentée à travers un détecteur d'ultraviolets (313 nm) et son absorption a été enregistrée pour donner une série de crêtes. Le retrait des peptides de la résine et le clivage simultané des groupes protecteurs de la chaîne latérale ont été effectués en utilisant un mélange de acide trifluoroacétique/eau/égoutier (95:5:5) pendant 2-3 heures. La chromatographie des peptides protégés-Nδ a été réalisée en utilisant du carbone graphitisé (PGC 220-224 ; 150-180 pm, 100 $m^2$/g) dans une colonne de verre. HPLC a été effectué sur un système Waters en utilisant une colonne Prep aquapore ABI 10 C-18 taille de pore 300 A silicium sphérique 20 µm (10 mmDI x 250 mm) pour les séparations de préparation et une colonne ABI RP 18 aquapore DE 300 silicium sphérique 7 µm (4,6 mmDI x 220 mm) pour les séparations analytiques. On a utilisé un gradiant, comme spécifié entre parenthèses, entre le solvant A (0,1% TFA dans l'eau) et le solvant B (0,1% dans de l'acétonitrile). Le taux de débit était de 1 ml/mn pour le HPLC analytique et de 5 ml/mn pour le HPLC de préparation. L'élution des échantillons a été surveillé par absorption d'ultraviolets à 214 nm. Les analyses d'acides aminés ont été effectués sur un analyseur d'acides aminés LKB 4150 alpha après une hydrolyse en tube étanche dans de l'acide hydrochlorique en ébullition constante à 110°C pendant 18-36 heures.

Synthèse de Fmoc Gly Ser Met Val Leu Ser OH et Tbfmoc Gly Ser Met Val Leu Ser OH et comparaison de ses propriétés

a. Synthèse de Fmoc Gly Ser Met Val Leu Ser OH

Le peptide lié à la résine protégé Fmoc Ser (OtBu) Met Val Leu Ser (OtBu) (43) a été préparé sur un synthétiseur à peptides en utilisant une stratégie orthogonale. Fmoc a été utilisé pour la protection temporaire de la fonction aminée de chaque acide aminé et a été retiré avant chaque accouplement avec une solution de piperidine à 20% dans du DMF. La fonction de chaîne latérale de la sérine a été protégée avec le groupe t-butyl. Les procédures d'accouplement ont impliqué un anhydride symétrique suivi d'un accouplement d'ester HOBt. De l'anhydride acétique et de la pyridine ont été utilisés pour acétyler toute fonction aminée non réagie. La résine Merrifield a été employée avec le groupe p-alkoxybenzylalcool comme lien.

Fmoc Gly Ser Met Val Leu Ser OH (44) a été synthétisé à partir du peptide lié à la résine (43) via les séries de réactions soulignées ci dessous

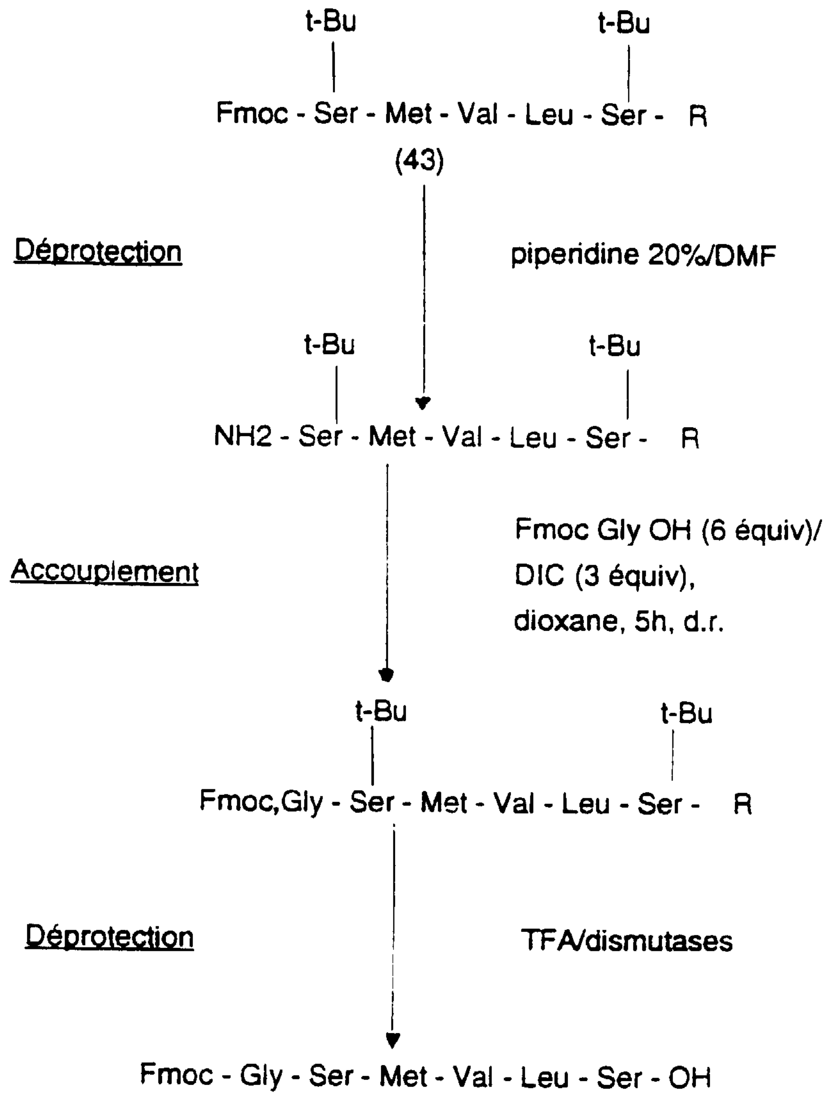


(43)

(44)

Après déprotection du groupe Fmoc dans des conditions basiques le terminus-aminé du peptide a été accouplé dans du dioxane à Fmoc Gly OH <u>via</u> son anhydride symétrique (3 équiv) avec une efficacité d'accouplement de 88%. Le clivage de l'hexapeptide de la résine et le retrait des groupes t-butyl ont été exécutés en utilisant du TFA, et en présence de sulfure d'éthyle et de méthyle et d'anisole comme dismutases de cation. Après le retrait du solvant <u>in vacuo</u> le peptide a finalement été précipité avec de l'éther, filtré et séché. La purification en utilisant le HPLC a été effectuée en utilisant du dioxane où le peptide était soluble à environ 1,5 mg/l. Le HPLC de préparation a donné le peptide (44) en même temps qu'une trace de dérivé de sulfoxyde, comme indiqué par la spectrométrie de masse (MH+16+). L'identité du peptide a été confirmé par la spectrométrie de masse et l'analyse des acides aminés.

<u>N-9-Fluorénylméthoxycarbonylglycylsérylméthionyl-valylleucylsérine, Fmoic Gly Ser Met Val Leu Ser OH</u> (44)

Le peptide lié à la résine Fmoc Ser (OtBu) Met Val Leu Ser (OtBu) (130,5 mg, 0,05 mmol) a été homogénéisé par ultrasons dans une solution de piperidine à 20% dans du N,N-diméthylformamide (5 ml) pendant 20 mn, puis filtré et enfin bien lavé avec du N,N-diméthyl-formamide, du dichlorométhane et du dioxane. Fmoc Gly OH (89,2 mg, 0,3 mmol; 6 équiv.) a été dissous dans du dioxane (2 ml) ; du 1,3-diisopropylcarbodiimide (47 µl, 0,3 mmol ; 6 équiv.) a ensuite été ajouté et le mélange de réaction a été homogénéisé par ultrasons pendant 5 mn. Cette solution a ensuite été ajoutée au peptide lié à la résine gonflé au préalable dans du dioxane (1 ml). Le mélange de réaction a été homogénéisé par ultrasons pendant 4,5 heures. Le peptide lié à la résine a été filtré, lavé avec du dichlorométhane, de l'éther et séché (129,8 mg) fonctionnalité de la résine du produit en mmol/g : 0,334, pourcentage

chargement/accouplement de la résine: 88 ; 4A300 nm = 0,76 (peptide-résine : 2,52 mg). On a ajouté de l'acide trifluoroacétique (10 ml) à un mélange de peptide-résine (126,1 mg, 0,042 mmol) anisole (0,25 ml), sulfure d'éthyle et de méthyle(0.25 ml) et eau (0,25 ml). Le mélange de réaction a été homogénéisé par ultrasons pendant 2 heures. La résine a été filtrée, lavée avec de l'acide trofluoroacétique (2 x 1 ml) et du chloroforme (4 ml). Le solvant a été retiré in vacuo pour donner un résidu. Le peptide a été précipité sur addition d'éther, filtré, lavé avec de l'éther et séché (42,7 mg) ; (Trouvé: Ser2 1,90, Gly, 1,05, Val, 0,99, Met, 1,02, Leul, 1,01) ; m/z (FAB) 853, 837, 815 (MH+). HRMS 815.36488, C39H55N6O11S1 nécessite : 815.36942 () < 1 ppm.

HPLC : colonne RP 18, solvants : A (H2O/TFA (0,1%)), B (CH3CN/TFA (0,1%)) ;

**25 mn**

**conditions: A (90%) ------> A (10%) ;**

lambda = 229 nm ; AUFS = 0,2 ; taux de débit : 1 ml/mn ; injection : 25 µl (C = 0,4 mg/0,5 ml (dioxane)) ; délai de rétention : 14,2 mn.

b. Synthèse de Tbfmoc Gly Ser Met Val Leu Ser OH

Tbfmoc Gly Ser Met Val Leu Ser OH (45) a ensuite été préparé à partir du peptide lié à la résine (43) via une méthode similaire.

Tbfmoc Gly OH a été couplé dans du dioxane avec une efficacité d'accouplement de 80%. Le retrait du peptide de la résine et le clivage de t-butyl ont été effectués avec du TFA en présence des mêmes dismutases. le peptide a finalement été précipité avec de l'éther, filtré et séché (rendement à 69%).

Le peptide a été purifié comme avant. Une solution fraîche du peptide brut dans du dioxane a donné une crête sur le HPLC, mais en attente (30 mn) la même solution a donné deux crêtes correspondant au peptide souhaité et au dérivé de sulfoxyde. L'oxydation facile de méthionine au sulfoxyde peut être due à la présence de péroxydes dans le dioxane. Après isolation de ces deux peptides par le HPLC de préparation, ces assignations ont été confirmées par la spectrométrie de masse [MH+ et MH+16+) et l'analyse des acides aminés.

N-17-Tétrabenzo(a,c,g,i)fluorénylméthoxycarbonyl-glucylsérylméthionylvalylleucylsérine, Tbfmoc Gly Ser Met Val Leu Ser OH (45)

Le peptide lié à la résine Fmoc Ser (OtBu) Met Val Leu Ser (OBUt) (65,3 mg, 0,025 mmol) a été homogénéisé par ultrasons dans une solution de piperidine à 20% dans du DMF (5 ml) pendant 20 mn. Le peptide lié à la résine a ensuite été filtré et bien lavé avec du DMF, du dichlorométhane et du dioxane. Tbfmoc Gly OH (74,5 mg, 0,15 mmol ; 6 équiv.) a été homogénéisé par ultrasons pendant 30 mn dans du dioxane (2 ml). Du 1,3-Diisopropylcarbodiimide (23,5 µl, 0,15 mmol; 6 équiv.) a ensuite été ajouté et le mélange de réaction a été homogénéisé par ultrasons pendant 5 mn. Cette solution a ensuite été ajoutée au peptide lié à la résine gonflé au préalable dans du dioxane (1 ml). Le mélange de réaction a été homogénéisé par ultrasons pendant 5 heures. La résine a été filtrée, bien lavée avec du dichlorométhane, du dioxane et de l'éther, puis séchée (58,3 mg) ; fonctionnalité de la résine du produit en mmol/g : 0,28 ; pourcentage de chargement/accouplement de la résine : 80 ; A364 nm = 0,43 (peptide lié à la résine : 0,85 mg). On a ajouté de "acide trifluoroacétique (5 ml) à un mélange de peptide lié à la résine (57,3 mg, 0,016 mmol), d'anisole (0,25 ml), de sulfure d'éthyle et de méthyle (0,25 ml) et d'eau (0,25 ml). Le mélange de réaction a été filtré et lavé avec de l'acide trifluoroacétique (1 ml) et du chloroforme (4 ml) et le solvant a été retiré in vacuo. L'homogénéisation Ju résidu dans de l'éther a donné le peptide souhaité sous la forme d'un solide jaune qui a été filtré, bien lavé avec de l'éther et séché (17,2 mg, 69%) ; (Trouvé : Ser2 1,8, Gly, 0,08, Val, 1,00, Met, 0,96, Leu, 1,00 ; m/z (FAB) 1053, 1037, 1015 (MH+). HRMS 1015.42761, C55H63N6O11S nécessite: 1015.42752 () < 1 ppm.

HPLC:colonne RP 18, solvants : A (H2O/TFA (0,1%)) ; B (CH3CN/TFA (0,1%)) ;

**22 mn.**

**conditions: A (75%) --------> A (5%) ;**

lambda = 229 nm ; AUFS = 1; taux de débit : 1 ml/mn.; injetion : 25 µl (C = 0,2 mg/0,2 ml (dioxane)) ; délai de rétention : 16,1 mn.

c. Comportement des dérivés de Tbfmoc et de Fmoc sur le PGC

La rétention à la fois de Fmoc Gly OH et de Tbfmoc Gly OH a été comparée à une colonne remplie de carbone graphitisé.

Une solution de Fmoc Gly OH (10,2 mg, 34,3 μmole) dans du dioxane a été chargée sur une colonne de 6 mm de diamètre remplie de PGC (1,5 g). 15 ml de dioxane ont suffi à éluer complètement le composé (sous la surveillance de t.l.c.). En contraste, quand Tbfmoc Gly OH (10,2 mg, 20,5 μmole) a été chargé dans les mêmes conditions, aucune partie de ce matériau n'a été éluée dans les premiers 35 ml. En fait, le volume total de dioxane nécessaire pour éluer le composé a été de 300 ml.

Le comportement sur une colonne de carbone graphitisé de Fmoc Gly Ser Met Val Leu Ser OH (44) a ensuite été comparé au dérivé de Tbfmoc (45). Pour augmenter le délai total de rétention, un mélange de dioxane/eau (2 : 1) a été utilisé pour éluer les deux peptides. Après chargement d'une solution d'hexapeptide de Fmoc (9,2 mg, 11,3 μmole) dans un mélange de dioxane/eau (2 : 1) sur la colonne, le peptide a été élué complètement avec 75 ml de mélange de solvant. En contraste, le dérivé de Tbfmoc (10,5 mg, 10,3 μmole) n'a pas été élué du tout dans les premiers 80 ml de solvant et n'a été par conséquent que très lentement élué de la colonne.

d. Déprotection du groupe Tbfmoc

La déprotection du groupe Tbfmoc du peptide a été effectuée alors qu'il était encore retenu sur la colonne de carbone graphitisé. La déprotection a été effectuée en utilisant de la piperidine à 20% dans un mélange de dioxane/eau (2 : 1). Dans ces conditions l'hexapeptide de Tbfmoc a été totalement déprotégé. Le peptide libre (HGly Ser Met Val Leu Ser OH (46)) a été élué et a été surveillé par t.l.c. en utilisant de la ninhydrine pour révéler la fonction aminée. L'isolation du produit qui en a découlé a été simple ; la piperidine et les solvants ont été retirés in vacuo et le peptide a été précipité avec de l'éther, filtré et séché (85%). La spectrométrie de masse a montré à la fois la présence du peptide (MH+) et celle du dérivé de sulfoxyde (MH+16+). L'analyse des acides aminés a confirmé la composition du peptide.

Glycylsérylméthionylvalylleucylsérine, H Gly Ser Met Val Leu Ser OH (46)

Une solution de Tbfmoc Gly Ser Met Val Leu ser OH (29,2 mg, 28,8 μmol) dans un mélange de dioxane et d'eau (2 : 1; 15 ml) a été chargée sur une colonne de 9 mm de diamètre remplie de carbone graphitisé (4 g). La colonne a ensuite été éluée avec un mélange de dioxane et d'eau (2 : 1; 60 ml). La déprotection a été effectuée en éluant la colonne avec de la piperidine à 20% dans un mélange de dioxane et d'eau (2 : 1; 20 ml). Les fractions contenant le peptide (surveillées par t.l.c., Rf = 0 (MeOH/CHCl3/CH3COOH (1 : 9 : 0.5) (en utilisant de la ninhydrine) ont été et évaporées pour donner un résidu. Le peptide a été précipité avec de l'éther, filtré et séché (14,5 mg, 85%) ; (Trouvé : Ser2 1,92, Gly1 0,98, Val1 1,03, Met1 0,87, Leu1 1,02 ; m/z (FAB) 609, 593 (MH+). HRMS 593.29689, C24H45O9N6S nécessite : 593.29685 () < 1 ppm.

Enfin, le tétrabenzofluorène oléfine ou son adduct de piperidine, les sous-produits de l'étape de déprotection, ont été retirés de la colonne par élution avec du dioxane chaud.

EXEMPLE 7

Synthèse de Fmoc Ubiquitine (54-76)OH et Tbfmoc Ubiquitine (53-76)OH et comparaison de leurs propriétés

a. Synthèse de Fmoc Ubiquitine (54-76) OH

Le peptide lié à la résine Fmoc ubiquitine (54-76) (47) a été préparé sur un synthétiseur de peptides dans les conditions décrites dans l'Exemple (a). La plupart des procédures d'accouplement ont impliqué un anhydride symétrique suivi d'un accouplement d'ester HOBt dans un mélange de DMF/dioxane (1 : 1) comme solvant.

b: Synthèse de Tbfmoc Ubiquitine (53-76) OH

Le Tbfmoc Ubiquitine (53-76) OH (48) a été synthétisé à partir du peptide lié à la résine (47) comme décrit ci-dessous.

Fmoc ubiquitine* (54-76)- R
(47)

Déprotection piperidine 20%/DMF

NH2 ubiquitine* (54-76)- R

Accouplement Tbfmoc Gly OH/DIC/HOBt

Tbfmoc ubiquitine* (53-76) OH R

Déprotection TFA/dismutases

Tbfmoc ubiquitine* (53-76) OH
(48)

* : chaînes latérales d'acide aminé protégées.

Le peptide lié à la résine Fmoc ubiquitine (54-76 a été initialement traité avec un mélange d'anhydride acétique/pyridine (1 : 1) afin d'amorcer toute fonction aminée. Après déprotection dans des conditions basiques, le peptide a été couplé dans du dioxane à Tbfmoc Gly OBt ester (4 équiv), généré in situ à partir de DIC et de HOBt, avec une efficacité d'accouplement de 88%. Après un clivage final utilisant le TFA en présence d'anisole et de thioanisole comme dismutases de cation, le peptide Tbfmoc ubiquitine (48) a été précipité avec de l'éther (rendement à 96%).

N-(1 7-Tétrabenzo(a,c,g,i)fluorénylméthoxycarbonyl) glycylarginylthréonylleucylséryllaspartyltyrosyl-asparagylisoleucylglutaminyllysylglutamylsérylthréonylleucylhistidylleucylvalylleucyla rginylleucylarginyl-glycylglycine, Tbfmoc-ubiquitine (53-76) OH (48)

De l'anhydride acétique (21,4 él, 226, µmol ; 10 équiv.) et de la pyridine (18,2 µl, 226 µmol ; 10 équiv.) ont été ajoutés au peptide lié à la résine (150 mg, 22,65 µmol) Fmoc-ubiquitine (54-76) dans du DMF (1 ml). Le mélange de réaction a été homogénéisé par ultrasons pendant 30 mn. Après filtration et lavage avec du DMF et de l'éther, le peptide lié à la résine a été homogénéisé par ultrasons pendant 15 mn dans une solution de piperidine à 20% dans du DMF (25 ml). Le peptide lié à la résine a ensuite été filtré et bien lavé avec du DMF et de l'éther. Tbfmoc Gly OH (45 mg, 0,09 mmol ; 4 équiv.) a été homogénéisé par ultrasons pendant 30 mn. dans du dioxane (1,5 ml) jusqu'à dissolution complète. Du 1,3-diisopropylcarbodiimide (14 µl, 0,09 mmol 4 équiv.) et du 1-hydroxybenzotriazole (12,2 mg, 0,09 mmol ; 4 équiv.) ont été ajoutés à cette solution. Le mélange de réaction a été homogénéisé par ultrasons pendant 2 mn. cette solution a ensuite été ajoutée au peptide lié à la résine gonflé au préalable pendant 30 mn. dans du dioxane (1,5 ml). Après sonication pendant 3,5 heures, le peptide lié à la résine a été filtré, lavé avec du dioxane et de l'éther et séché jusqu'au lendemain dans un dessiccateur sous vide (148,1 mg).

Efficacité de substitution : le peptide lié à la résine Tbfmoc (2,1 mg) a été homogénéisé par ultrasons pendant 30 mn. dans une solution de triéthylamnie à 20% dans du DMF (10 ml). L'absorption spécifique à 364 nm a été enregistrée et les résultats suivants en ont été dérivés : fonctionnalité de la résine du produit en mmol/g : 0,133; pourcentage de chargement/accouplement de la résine : 88 ; A364 nm = 0,505 (peptide lié à la résine : 2,1 mg).
De l'eau (75 µl), du thioanisole (75 µl), de l'anisole (75 µl) et de l'acide trifluoroacétique (3 ml) ont été ajoutés au peptide-Tbfmoc lié à la résine (146 mg). Le mélange de réaction a été homogénéisé par ultrasons pendant 2,5 heures. La résine a été filtrée, lavée avec du TFA ( 2 x 0,5 ml) et du chloroforme (2 x 1 ml), et séchée (24 mg). Le filtrat a été évaporé sous pression réduite pour donner un résidu. L'homogénéisation dans l'éther a donné le peptide brut sous la forme d'un solide jaune qui a été refroidi jusqu'au lendemain, filtré et séché (68,5 mg). Une suspension de Tbfmoc-ubiquitine (53-76) brut (12 mg) dans CH3CN/H2O (1 : 1; 0,1% TFA ; 12 ml) a ensuite été homogénéisée par ultrasons pendant 2 heures jusqu'à ce que la dissolution complète se soit produite. La purification de ce produit a finalement été réalisée par un HPLC de préparation en utilisant une colonne C-18 à phase inversée (10 x 250 mm) avec un gradiant de A : B, 20 à 80% B pendant 25 mn et détection d'ultraviolets à 214 nm, pour rendre du Tbfmoc ubiquitine (53-76) pure après lyophilisation sous forme de poudre blanche (4,5 mg) ; analyse des acides aminés : Asx2 2,14, Thr2 1,94, Ser2 1,87, Glx2 2,38, Gly3 2,73, Val1 1,14, Ile1 0,94, Leu5 5,02, Tyr1 0,94, His1 1,01, Lys1 0,94, Arg3 2,94 ; m/z (FAB) 3147,4 (MH+), HRMS 3149.64063, C149H219N38O38 nécessite : 3149.64048 () <
1 ppm ;

25 mn.
HPLC (A:B, A (90%) -------> A (10%) ;

214 nm ; C = 0,2 mg/0,2 ml (A), injection : 25 µl) Rt = 18,2 mn.

c. Comportement de N-Acétyl-Ubiquitine (54-76) OH et de Tbfmoc Ubiquitine (53-76) OH sur le PGC

N-Acétyl-Ubiquitine (54-76) OH (49) a été préparé à partir du peptide lié à la résine (47) par les réactions suivantes. La déprotection du groupe Fmoc à partir du peptide en utilisant de la piperidine à 20% dans du DMF a donné le peptide avec un groupe aminé N-terminal libre, qui a été ensuite acétylé avec un mélange d'anhydride acétique/pyridine (1 : 1) (100 équiv). Le retrait du peptide de la résine et le clivage des groupes protecteurs des chaînes latérales ont été effectués en utilisant du TFA en présence d'anisole et thioanisole comme dismutases. Une précipitation consécutive avec de l'éther a donné N-acétyl -ubiquitine (54-76) OH (49) brut.

N-(Acétyl)arginylthrèonylleucylsérylaspartyltyrosyl-asparagylisoleucylglutaminyllysylglutamylsérylthréonyl-histidylleucylvalylleucylarginylleucylarginylglycylglycine, ND-acétyl-ubiquitine (54-76) OH (49)

Le peptide lié à la résine Fmoc-ubiquitine (54-76) (100 mg, 0,0151 mol) a été homogénéisé par ultrasons pendant 30 mn dans une solution de piperidine à 20% dans du DMF (25 ml) puis filtré, et enfin bien lavé avec du DMF et de l'éther. De l'anhydride acétique (143 µl, 1,51 mmol ; 100 équiv.) et de la pyridine (122 µl, 1,51 mmol ; 100 équiv.) ont été ajoutés au peptide lié à la résine gonflé au préalable pendant 30 mn dans du DMF (1 ml). Le mélange de réaction a été homogénéisé par ultrasons pendant 1 heure. Le peptide lié à la résine a été filtré et lavé avec du DMF et de l'éther. de l'eau (50 µl), du thioanisole (50 µl), de l'anisole (50 µl) et de l'acide trifluoroacétique (2 ml) ont été ajoutés au peptide-acétyl-N lié à la résine. Le mélange de réaction a été homogénéisé par ultrasons pendant 2,5 heures. La résine a été filtrée, lavée avec du TFA ( 2 x 0,5 ml) et du chloroforme (2 x 0,5 ml). Le solvant a été retiré in vacuo, L'homogénéisation dans l'éther a donné le produit sous forme d'un précipité blanc qui a été refroidi jusqu'au lendemain, filtré et séché (43,6 mg). La purification de ce peptide (23,4 mg) a été réalisée par HPLC de préparation en utilisant la même colonne C-18 à phase inversée avec un gradiant de A : B, 10 à 60% B pendant 16 mn. et la détection d'ultraviolets à 214 nm, pour donner un N-acétyl-ubiquitine (54-76) OH pur sous forme d'un solide blanc après lyophilisation (9,7 mg) ; analyse des acides aminés : Asx2 1,98, Thr2 1,82, Ser2 1,69, Glx2 2,33, Gly2 2,36, Val1 1,14, Ile1 0,91, Leu5 5,06, Tyr1 0,85, His1 1,01, Lys1 0,98, Arg3 2,87 ; m/z (FAB) 2712,1 (MH+), HRMS 2712.49903, C118H200N37O36 nécessite : 2712.49891 () < 1 ppm ;

25 mn.
HPLC (A : B, A (90%) -------> A (10%) ;

214 nm ; C = 0,2 mg/0,2 ml (A), injection: 25 µl) Rt = 13,2 mn.

Le comportement à la fois des peptides N-acetyl et Tbfmoc ubiquitine sur une colonne de PGC a ensuite été examiné.

Une solution contenant 5 mg de chaque peptide a été chargée sur la colonne qui a ensuite été éluée en utilisant différents mélanges de solvants. Après avoir regroupé les fractions, le solvant a été retiré in vacuo et le résidu

obtenu a été de nouveau dissous dans du solvant frais avant analyse au HPLC.

Les meilleurs résultats ont été obtenus quand un mélange de CH3CN/H2O (1 : 1) a été utilisé. Dans ces conditions, le peptide-acétyl-N a été complètement élué par 30 ml du mélange, alors que le peptide Tbfmoc était retenu.

La séparation chromatographique totale de ces peptides très proches l'un de l'autre a clairement démontré l'efficacité de la méthode et son potentiel pour la purification des peptides synthétisés par méthodologie de phase solide, dans la mesure où l'on apporte une attention toute particulière au choix des solvants.

d. Purification de l'Ubiquitine (53-76) OH

Une simple élution chromatographique sur une colonne remplie de carbone graphitisé a été utilisée comme première étape de purification (comme décrit en (c) ci-dessus). De l'ubiquitine Tbfmoc (53-76) OH (48) brute dissoute dans un mélange de CH3CN/H2O (1:1; 0,5% TFA) a été chargée sur une colonne remplie de PGC (50 x masse du peptide). 50 ml d'un mélange du même solvant ont été nécessaires pour éluer complètement les principales impuretés (probablement N-acétyl ubiquitine (55-76) et de l'ubiquitine (54-76) non réagie). Aucun des peptides Tbfmoc n'a été élué même après rinçage de la colonne avec 50 ml supplémentaires de CH3CN/H2O (1:1). La Déprotection du groupe Tbfmoc a été effectuée en utilisant de la piperidine à 20% dans un mélange de CH3CN/H2O (1 : 1) et seul le peptide souhaité, l'ubiquitine (53-76) OH, a été élué. Après le retrait du solvant in vacuo, le peptide a finalement été précipité avec de l'éther. Une purification consécutive par HPLC de préparation a donné de l'ubiquitine (53-76) OH pure (50) en rendement total de 15%.

Glycylarginylthréonylleucylséryiaspartyltyrosylasparagyl-isoleucylglutaminyllysylglutamylsérylthréonylleucylhistidyl-leucylalylleucylarginylleucylarginylglycylglycine. ubiquitine (53-76) OH (50)

Une solution de Tbfmoc-ubiquitine (53-76 OH (30 mg ; peptide brut) dans un mélange de CH3CN/H2O (1 : 1; 0,5% TFA ; 30 ml) a été homogénéisée par ultrasons pendant 30 minutes jusqu'à dissolution complète et chargée sur une colonne de verre de 5 mm de diamètre remplie de carbone graphitisée (1,4 g ; PGC 220-224; 150-180 µm 100 m2/g; longueur après remplissage : 140 mm). La colonne a été d'abord éluée avec un mélange de CH3CN/H2O (1 : 1; 0,5% TFA ; 50 ml). Après lyophilisation un résidu (6,1 mg) a été obtenu, qui a donné une crête importante sur HPLC (Rt = 13 mn., N_-acétyl-ubiquitine (55-76) ou ubiquitine (54-76)). La colonne a ensuite été éluée avec un mélange de CH3CN/H2O (1 : 1; 50 ml). Un résidu (0,4 mg) a été obtenu après lyophilisation; on a découvert par HPLC qu'il contenait des impuretés. La déprotection a été effectuée en utilisant une solution de piperidine à 20% dans un mélange de CH3CN/H2O (1 : 1; 50 ml). Le solvant a été retiré in vacuo pour donner un résidu qui a été homogénéisé dans l'éther, filtré et séché (11 mg). Ce peptide brut (11 mg) a finalement été purifié par HPLC de préparation (colonne C-18 à phase inversée (10 x 250 mm); gradiant (A : B), 10 à 60% B pendant 25 mn; détection à 214 nm pour donner de l'ubiquitine (53-76) OH sous la forme d'un solide blanc après lyophilisation (4,5 mg, 15%); analyse des acides aminés : Asx2 2,04, Thr2 1,88, Ser2 1,67, Glx2 2,27, Gly3 3,37, Val1 1,01, Ile1 0,90, Leu5 4,99, Tyr1 0,93, His1 1,01, Lys1 0,96, Arg3 2,95; m/z (FAB) 2727,8 (MH+), HRMS 2727.50986, C118H201N38O36 nécessite : 2227.50981 () < 1 ppm ;

$$\text{HPLC (A : B, A (90\%)} \xrightarrow{\text{25 mn.}} \text{A (10\%) ;}$$

214 nm ; C = 0,4 mg/0,4 ml (A), injection : 25 µl) Rt = 12,6 mn.

Les identités à la fois du peptide Tbfmoc ubiquitine (53-76) OH et du peptide ubiquitine (53-76) OH ont été établies par spectrométrie de masse à haute résolution et analyse des acides aminés.

EXEMPLE 8

Synthèse et purification de l'Ubiquitine (35-76)

La purification de ce peptide n'a pas posé de problème et a été effectuée comme pour le plus petit fragment d'ubiquitine (50) ; Tbfmoc ubiquitine (35-76) OH (52) a été préparé en couplant in situ Tbfmoc Gly OBt à l'ubiquitine du peptide lié à la résine (36-76) (rendement à 75%). Après chromatographie sur une colonne de PGC et HPLC de prépartion, le peptide souhaité (51) a pu être obtenu.

L'authenticité de (51) a également été confirmée par la spectrométrie de masse et l'analyse des acides aminés, et sa pureté par HPLC analytique.

N-(17Tétrabenzo(a,c,g,i)fluorénylméthoxycarbonyl)) glycylisoleucylprolylprolylaspartylglutaminylglutaminyl-arginylleucylisoleucylphénylalanylalanylglycyllysyl-glutaminylleucyglutamylaspartylglycylarginylthréonylleucyl-sérylaspartyltyroasparagylisoleucylglutaminyllysylglutamyl-sérylthréonylleucylhistidylleucylvalylleucylarginylleucyl-arginylglucylglycine, Tbfmoc ubiquitine (35-76) OH (52)

De l'anhydride acétique (14,8 µl, 157,6 µmol ; 20 équiv) et de la pyridine (12,8 µl, 157,6 µmol ; 20 équiv) ont été ajoutés au peptide lié à la résine Fmoc ubiquitine (36-76) (109,3 mg, 7,88 µmol) dans du DMF (1 ml). Le mélange de réaction a été homogénéisé par ultrasons pendant 1 heure. Après filtration et lavage avec du DMF et de l'éther, le peptide lié à la résine a été homogénéisé par ultrasons pendant 15 minutes dans une solution de piperidine à 20% dans du DMF (25 ml). Le peptide lié à la résine a ensuite été filtré et bien lavé avec du DMF et de l'éther. Tbfmoc GlyOH (19,6 mg, 39,4 µmol ; 5 équiv) a été homogénéisé par ultrasons pendant 30 Minutes dans du dioxane (1 ml). On a ajouté à cette solution du 1,3-diisopropylcarbodiimide (6,2 µl, 39,4 µmol ; 5 équiv) et du 1-hydroxybenzotriazole (5,3 mg, 39,4 µmol ; 5 équiv). Le mélange de réaction a été homogénéisé par ultrasons pendant 5 minutes. Cette solution a ensuite été ajoutée au peptide lié à la résine gonflé au préalable dans du dioxane (1 ml) pendant 1 heure. Le mélange de réaction a été homogénéisé par ultrasons pendant 19 heures. Le peptide lié à la résine a été filtré, lavé avec du dioxane, du dichlorométhane et de l'éther et finalement séché pendant 48 heures dans un dessiccateur sous vide (102 mg).

Efficacité de substitution : le peptide lié à la résine Tbfmoc (2,9 mg) a été homogénéisé par ultrasons pendant 30 minutes dans une solution de triéthylaminé à 20% dans du DMF (10 ml). L'absorption spécifique à 364 nm a été enregistrée et les résultats suivants en ont été dérivés: A = 0,265 ; fonctionnalité de la résine du produit en µmol/g: 50,51; pourcentage de chargement/accouplement de la résine : 70. L'accouplement a ensuite été répété dans les mêmes conditions (4 équivalents de chaque Tbfmoc GlyOH, 1,3-diisopropylcarbodiimide et 1-hydroxybenzotriazole ; délai de réaction : 3,5 heures). Le peptide lié à la résine a été filtré, lavé avec du dioxane, du dichlorométhane et de l'éther et enfin séché jusqu'au lendemain dans un dessiccateur sous vide (99,5 mg).

Efficacité de substitution : A = 0,265 (peptide lié à la résine : 2,7 mg) ; fonctionnalité de la résine du produit en µmol/g: 54,26 ; pourcentage de chargement/accouplement de la résine : 75.

De l'eau (75 µl), du thioanisole (50 µl) et du TFA (2,1 ml) ont été ajoutés au peptide-Tbfmoc lié à la résine (96,9 mg). Le mélange de réaction a été homogénéisé par ultrasons pendant 2,5 heures. La résine a été filtrée, lavée avec du TFA (2 x 0,5 ml) et du CHCl3 (2 x 1 ml), et séchée (19,1 mg). Le solvant a été retiré in vacuo pour donner un résidu. L'homogénéisation dans de l'éther d'éthyle a donné le peptide brut sous forme d'un solide jaune qui a été refroidi jusqu'au lendemain, bien lavé avec de l'éther et finalement séché (49,2 mg).

25 mn.

HPLC (A : B ; A (90%) ——> A '10%;

214 nm ; C = 0,5 mg/0,5 ml (A/B (1 : 1)) ; injection 30 µl) Rt = 19,2 mn.

Glycylisoleucylprolylprolylaspartylglutaminylglutaminyl-arginylleucylisoleucylphénylalanylalanylglycyllysyl-glutaminylleucylglutamylaspartylglycylarginylthréonyl-leucylsérylaspartyltyrosylasparagylisoleucylglutaminyl-lysylglutamylsérylthréonylleucylhistidylleucylyalyl-leucylarginylleucylarginylglycylglycine, ubiquitine (35-76) OH (51)

Une suspension de Tbfmoc-ubiquitine (53-76) brut OH (30 mg) dans un mélange de CH3CN/H2O (1 : 1; 0,5% TFA, 25 ml) a été homogénéisée par ultrasons pendant 20 minutes jusqu'à dissolution complète) et chargée sur une colonne en verre de 5 mm de diamètre remplie de carbonne graphitisé (1,5 g ; PGC 220-224 ; 150-180 µm ; 100 $m^2/g$; longueur active : 15 cm). La colonne a d'abord été éluée avec un mélange de CH3CN/H2O (1 : 1; 0,5% TFA ; 50 ml). Après lyophilisation de l'éluant, un résidu (2 mg) a été obtenu qui a donné une crête large sur HLPC (Rt = 13,8 mn (impuretés)). La colonne a ensuite été éluée avec un mélange de CH3CN/H2O (1 : 1; 50 ml). Un solide blanc (4,5 mg) a été obtenu après lyophilisation ; on a découvert par HPLC qu'il contenait également des impuretés (crête large, Rt = 14,1 mn). La colonne a été de nouveau éluée avec 50 ml d'une mixture de CH3CN/H2O (1 : 1) et un résidu (0,3 mg) a été obtenu après lyophilisation (Rt = 14,2 mn). La déprotection a été effectuée en utilisant une solution de piperidine à 20% dans un mélange de CH3CN/H2O (1 : 1;
50 ml) et a été suivie d'un élution de la colonne avec CH3CN/H2O (1 : 1; 50 ml). Le solvant a été retiré in vacuo pour donner un résidu marron. Du diéthyl éther a été ajouté et le précipité obtenu a été refroidi jusqu'au lendemain, filtré et bien lavé avec de l'éther, et enfin séché (11,7 mg). Le peptide brut (24,8 mg) a finalement été purifié par HPLC de préparation (colonne C-18 à phase inversée (10 x 250 nm) ; gradiant (A: B), 20 à 60% B pendant 22 mn ; détection à 214 nm) pour donner de l'ubiquitine (35-76) OH pure sous la forme d'un solide blanc après lyophilisation (4,4 mg) ;

analyse des acides aminés ; Asx4 3.98. ThR2 1.83. Ser2 1.75. Glx6 6.67. Pro2 1.91. 0,96, Val1 1,01, Ile3 2,99, Leu7 7,00, Tyrl 0,94, Phe1 1,04, His1 1,11, Lys2 1,91, Arg4 3,81; m/z (FAB) 4734,2 (MH+),
HRMS 4734.57186, C208H344N63063 nécessite: 4734.57161 ( ) < 1 ppm ;

25 mn.
**HPLC (A : B, A (90%) -------> A (10%) ;**

214 nm ; C = 0,1 mg/0,1 ml (A), injection : 25 µl) Rt = 13,8 mn.

EXEMPLE 9

Synthèse de Tbfmoc-L-Phe OH

La fonction aminée libre de l'hydrochlorure ester tert-butyl L-phénylalanine a été initialement libérée avec du triéthylaminé. L'aminé en excès de 20% a ensuite réagi avec le carbonate mélangé (41) en présence de N,N'-diméthylaniline. Après le retrait du groupe t-butyl avec le TFA, le composé (53) a été obtenu.

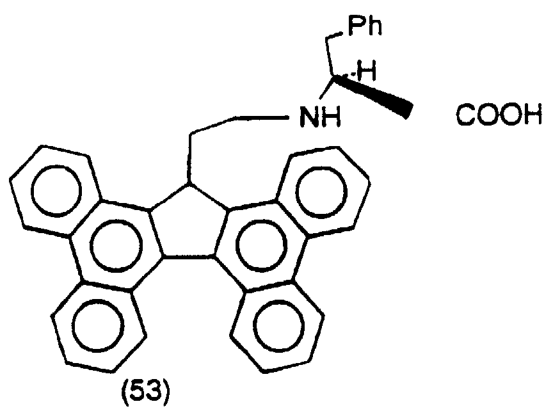

(53)

La formation de (53) peut être rationalisée par l'élimination β du carbonate mélangé (41), l'addition 1,2 de l'ester aminé sur l'oléfine en résultant (38) et enfin le clivage de l'ester t-butyl.

N-17-Tétrabenzo(a,c,g,i)fluorénylméthyl-L-phénylalanine, Tbfm-L-Pne OH (53)

Une solution de N-17-tétrabenzo(a,c,g,i) fluorénylméthyl-L-phénylalanine tert-butyl ester (421,1 mg, 0,702 mmol) dans de l'acide trifluoroacétique (3,6 ml) et de l'eau (0,2 ml) a été homogénéisé par ultrasons pendant 3,5 heures à température ambiante. Le solvant a été retiré in vacuo pour donner un résidu marron. L'homogénéisation dans du diéthyléther a donné le composé titre sous la forme d'un solide jaune qui a été refroidi jusqu'au lendemain, filtré, lavé avec de l'éther et finalement séché (352,7 mg, 92%) ; p.f. 178-180C ; (Trouvé: C, 85,2 ; H, 5,47 ; N, 2,57 C39H29NO2 nécessite 86,2 ; H, 5,37 ; N, 2,59%) ; (_)D20 -40,4° (C = 1, TFA); t.l.c. Rf (H) 0,36, Rf (T) 0,62 ; pmax (KBr) 3090, 3060, 3030 (CH extension, aryl), 1620 (COO-), 1500 (anneaux aromatiques), 1240, 1190, 1040 (CO extension), 750, 725, 700 (CH déformation hors plan) cm-1; lambda max 384 (18868), 369 (21267), 304 (52447), 292 (41894), 264 (78991), 255 (86346) nm ; δC (TFA, 50 MHz) 169,5 (CO, acide), 137,9, 136,9, 136,1, 134,2, 131,8, 131,4, 130,5, 129,8, 126,3, 126,1, 125,8 (C aromatiques quatemaires) 128,7, 127,9, 127,2, 127,0, 126,8, 124,8, 123,44, 123,34, 123,29, 122,2 (CH aromatiques), 62,2 (_CH), 50,9 (CH2), 43,2 (CH), 34,2 (β CH2) ; m/z (FAB) 544 (MH+), 379. HRMS 544.22762, C39H30NO2 nécessite : 544.22764 ( ) < 1 ppm.

Comme l'acétate de la glycine a eu du succès dans la synthèse de Tbfmoc Gly OH, cette méthode est également utilisée dans ce cas ; ainsi le sel d'acétate d'ester tert-buytl L-phénylalanine a été préparé.

Le sel d'hydrochlorure de l'ester d'acide aminé a été neutralisé avec de la triéthylamine et l'Et3N.HCl précipité engendré a été filtré. Suite au retrait du solvant, le résidu obtenu a été de nouveau dissous dans de l'acide acétique avant lyophilisation. La réaction consécutive avec le carbonate mélangé (41) en présence de deux équivalents de N, N'-diméthylaniline a donné Tbfmoc-L-Phe OtBu (54) en rendement à 46% après purification par chromatographie flash et recristallisation. Le clivage final de l'ester t-butyl en utilisant un mélange de TFA/H2O (95:5) a donné gTbfmoc-L-Phe OH (55) en excellent rendement.

N-17-tétrabenzo(a,c,g,i)fluorénylméthoxycarbonyl-L-phénylalanine tert-butyl Ester, Tbfmoc Phe OtBu (54)

On a ajouté de la triéthylamine (68,5 µl, 0,491 mmol) à une suspension d'hydrochlorure d'ester tert-butyl L-phénylalanine (126,7 mg, 0,491 mmol) dans de l'acétate d'éthyl (6 ml). Le mélange de réaction a été remué pendant 2,5 heures à la température ambiante. L'hydrochlorure de triéthylamine précipité a été filtré, lavé avec de l'acétate d'éthyl (2 x 0,5 ml) et séché (65,3 mg, 97%). Le filtrat a été évaporé pour donner un résidu qui a été de nouveau dissous dans de l'acide acétique. Après lyophilisation, le sel d'acétate a été obtenu sous forme d'un solide blanc (83,9 mg, 61%).

On a ajouté du N,N'-diméthylaniline (63 µl, 0,497 mmol ; 2 équiv) à une solution de 17-tétrabenzo(a,c,g,i) fluorénylméthyl-para-nitrophényl carbonate (139,4 mg, 0,248 mmol) et d'acétate d'ester de tert-butyl L-phénylalanine (83,9 mg, 0,298 mmol ; 1,2 équiv). Le mélange de réaction a été remué à température ambiante sous azote pendant 120 heures. Après addition d'eau (10 ml) et acidification avec KHSO4 (2M) à pH=1, le mélange de réaction a été extrait avec du dichlorométhane (3 x 20 ml). Les phases organiques combinées ont été lavées avec de l'eau (2 x 15 ml) à pH 6-7 et séchées sur MgSO4. Après filtration, le solvant a été évaporé sous pression réduite pour donner une huile orange. Après purification par chromatographie éclair en utilisant du toluène comme éluant, les fractions contenant le matériau de Rf=0,04 ont été évaporées pour donner un solide jaune. La recristallisation de l'éther/pétrole (p.l; 40-60°C) a donné le composé titre sous forme d'un solide jaune pâle qui a été filtré, lavé avec du pétrole et finalement séché (73,9 mg, 46%) ; p.f. 158-162°C (dec) ; t.l.c. Rf (C) 0,04, Rf (H) 0,84 ; pmax (KBr) 3280 (NH extension), 3060, 3030 (CH extension, aryl), 2980, 2930 (CH extension, alcoyl), 1735 (CO, ester), 1715 (CO, uréthane), 1680 (amide 1), 1610 (anneaux aromatiques), 1545 (amide II), 1500 (anneaux aromatiques) 1440 (déformations CH, alcoyl), 1390, 1370 (CH3, déformations symétriques), 1290, 1255, 1220, 1155, 1050 (CO extension), 850, 750, 720, 700 (déformation CH hors plan) cm-1; m/z (FAB) 643 (M+), 379. HRMS 643.27225, C44H37NO4 nécessite : 643.27224 ( ) < 1 ppm.

N-17-Tétrabenzo(a,c,g,i)fluorénylméthoxycarbonyl-L-phénylalanine, Tbfmoc-L-Phe OH (55)

Une solution de N-17-tétrabenzo(a,c,g,i)fluorényl-méthoxycarbonyl-L-phénylalanine tert-butyl ester (68,2 mg, 0,106 mmol) dans de l'acide trifluoroacétique (950 µl) et de l'eau (50 µl) a été homogénéisé par ultrasons pendant 2,5 heures. Le solvant a été retiré in vacuo pour donner un résidu violet. Un mélange de diéthyl éther et de pétrole p.i. 40-60°C (1 : 1) a été ajouté. Le précipité obtenu a été refroidi jusqu'au lendemain, filtré, lavé avec du pétrole et enfin séché dans un dessiccateur sous vide sur P2O5 pour donner le composé titre (57,6 mg, 93%) ; p.f. 137-140°C ; (d) D20 -50,8 (C = 0,25, CH2Cl2) ; t.l.c. : Rf (H) 0,43, Rf (I) 0,89; pmax (KBr) 3410 (NH extension), 3060, 3030 (CH extension, aryl), 2960, 2860 (CH extension, alcoyl), 1715 (CO, acide et uréthane), 1610, 1500 (chainons aromatiques), 1435, 1420 (déformations CH, alcoyl), 1340 (courbure OH), 1215, 1050 (CO extension), 750, 730, 700 (déformation CH hors-plan) cm-1; lambda max 384 (15794), 368 (16896), 302 (39670), 290 (32324), 262 (59505), 254 (64647) nm ; δH (CDCl3, 200 MHz) 8,80-8,58 (6H, m, aromatique), 8,30-8,19 (2H, m, aromatique), 7,65-7,54 (8H, m, aromatique), 7,19-7,07 (5H, m, aromatique (Phe)), 5,13 (1H, t apparent, CH), 4,98 (1H, d, 3J = 8,4 Hz, NH), 4,79-4,64 (2H, m, Ha (CH2) et CH (Phe)), 4,30-4,24 (1H, m, Hb (CH2)), 3,08 (2H, m, β CH2 (Phe)); δC (CDCl3, 90 MHz), 175,8 (CO, ACIDE), 155,8 (CO, uréthane), 142,6, 141,0, 136,8, 136,5, 135,3, 131,5, 130,3, 130,1 (C aromatiques quaternaires), 129,0, 128,6, 127,4, 127,3, 127,1, 126,8, 126,6, 125,9, 125,7, 125,5, 125,1, 124,9, 124,8, 123,4, 123,1, 123,0 (CH aromatiques), 69,2 (CH2), 54,4 (_CH), 47,4 (CH), 37,4 (β CH2); m/z (FAB) 587 (M+), 379. HRMS 588 21744 C40H30NO4 nécessite: 588.21747 () < 1 ppm.

## Revendications

1. Procédé caractérisé par le fait qu'il comporte l'étape de lier physiquement à un matériau carboné une molécule dont au moins une fonction est protégée par un groupe de formule (I):

Ar-L-

où

Ar représente un système de cycles fusionnés substantiellement plan contenant au moins 4 cycles aromatiques, et
L représente un groupe contenant au moins un atome de carbone qui est capable de se lier à une fonction à protéger; et

où

ledit matériau carboné est choisi parmi le graphite ou ceux à surfaces graphitisées y compris le charbon activé.

**2.** Procédé selon la revendication 1, caractérisé par le fait que l'étape de lier physiquement à un matériau carboné est un passage du mélange de composés dans une chambre remplie dudit matériau carboné.

**3.** Procédé selon les revendications 1 et 2, caractérisé par le fait qu'il comporte en outre l'étape de protection d'au moins une fonction dans au moins un composé au moyen un groupe de formule (I).

**4.** Procédé selon les revendications 1 à 3, caractérisé par le fait que Ar contient au moins 6 cycles aromatiques.

**5.** Procédé selon les revendications 1 à 4, caractérisé par le fait que les cycles aromatiques sont hexagonaux.

**6.** Procédé selon les revendications 1 à 5, caractérisé par le fait que Ar ne contient aucun hétéro-atome.

**7.** Procédé selon les revendications 1 à 6, caractérisé par le fait que la formule (i) répond à la formule (II) suivante :

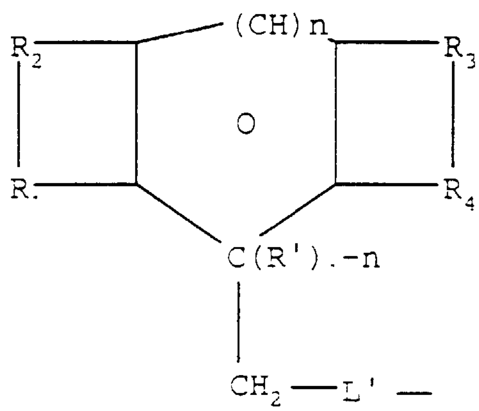

où n est égal à 0 ou 1;

O signifie que le cycle environnant est aromatique quand n est égal à 1 et non-aromatique quand n est égal à 0 ; R1 et R2, d'une part, et R3 et R4, d'autre part, sont choisis de façon à ce que chacun forme un système de cycles aromatiques fusionnés ensemble avec les atomes de carbone auxquels ils sont rattachés ; R' est un groupe alcoyle ou hydrogène ; et L' représente un lien direct ou un groupe capable de se lier à un groupe à protéger.

**8.** Procédé selon les revendications 1 à 6, caractérisé par le fait que la formule (I) répond à la formule (III):

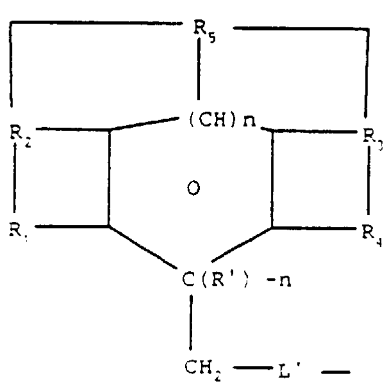

où

n est égal à 0 ou 1;
O signifie que le cycle environnant est aromatique quand n est égal à 1 et non-aromatique quand n est égal à 0 ; R1 et R2, d'une part, et R3 et R4, d'autre part, sont choisis de façon à ce que chacun forme un système de cycles aromatiques fusionnés ensemble avec les atomes de carbone auxquels ils sont rattachés ; R' est un groupe alcoyle ou hydrogène; et L' représente un lien direct ou un groupe capable de se lier à un groupe à protéger

et

où R5 est un groupe qui, ensemble avec les atomes auxquels il est rattaché, forme un ou plusieurs cycles supplémentaires.

9. Procédé selon les revendications 7 et 8, caractérisé par le fait que L'est -CO-, -O- CO-, -S, -O-, -(CH2)m-O- où m est de 1 à 6, ou simplement une liaison directe.

10. Procédé selon les revendications 1 à 8,caractérisé par le fait que la fonction de ladite molécule à protéger est un groupe alcool, thiol, acide, amine ou amide.

11. Procédé selon les revendications 1 à 10, caractérisé par le fait que le ladite molécule à protéger est un peptide naturel ou synthétique ou un acide aminé.

12. Utilisation comme groupe protecteur, d'un groupe ayant la formule suivante (I):

Ar-L-

où Ar représente un système de cycles fusionnés substantiellement plan contenant au moins 4 cycles aromatiques, et L représente un groupe contenant au moins un atome de carbone qui est capable de se lier à un groupe à protéger.

13. Utilisation selon la revendication 12, caractérisée par le fait que Ar contient au moins 6 cycles aromatiques.

14. Utilisation selon les revendications 13 et 14, caractérisé par le fait que les cycles aromatiques sont hexagonaux.

15. Utilisation selon les revendications 12 à 14, caractérisé par le fait que Ar ne contient aucun hétéro-atome.

16. Utilisation selon les revendications 12 à 15, caractérisé par le fait que où la formule (i) répond à la formule (II)

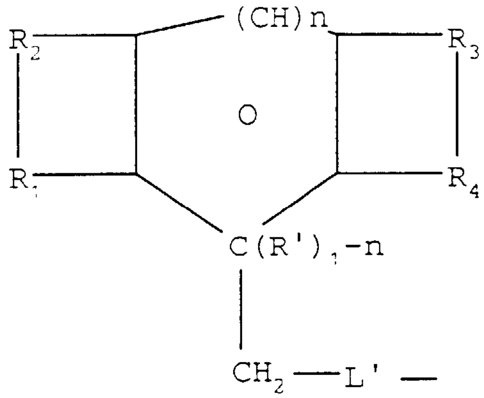

où n est égal à 0 ou 1;
O signifie que le cycle environnant est aromatique quand n est égal à 1 et non-aromatique quand n est égal à 0 ; R1 et R2, d'une part, et R3 et R4, d'autre part, sont choisis de façon à ce que chacun forme un système de

cycles aromatiques fusionnés ensemble avec les atomes de carbone auxquels ils sont rattachés ; R' est un groupe alcoyle ou hydrogène ; et L' représente un lien direct ou un groupe capable de se lier à un groupe à protéger.

**17.** Utilisation selon les revendications 12 à 15, caractérisé par le fait que la formule (I) répond à la formule (III):

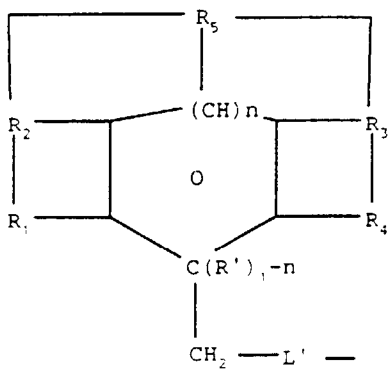

où

n est égal à 0 ou 1;
O signifie que le cycle environnant est aromatique quand n est égal à 1 et non-aromatique quand n est égal à 0 ; R1 et R2, d'une part, et R3 et R4, d'autre part, sont choisis de façon à ce que chacun forme un système de cycles aromatiques fusionnés ensemble avec les atomes de carbone auxquels ils sont rattachés ; R' est un groupe alcoyl ou hydrogène ; et L' représente un lien direct ou un groupe capable de se lier à un groupe à protéger

et où

R5 est un groupe qui, ensemble avec les atomes auxquels il est rattaché, forme un ou plusieurs cycles supplémentaires.

**18.** Composé utile pour la mise en oeuvre du procédé selon la revendication 1 , caractérisé par le fait qu'il comporte un groupe groupe de formule (I):

Ar-L-

où

Ar représente un système de cycles fusionnés substantiellement plan contenant au moins 6 cycles aromatiques, et
L représente un groupe contenant au moins un atome de carbone qui est capable de se lier à une fonction à protéger, rattaché, via le groupe L,.à un groupe partant ou à une molécule à protéger.

**19.** Composé selon la revendication 18, caractérisé par le fait que L est le groupe - CR'-L'-, où L' représente un lien direct ou un groupe capable de se lier à un groupe à protéger et R' est un groupe alcoyle, ayant de préférence jusqu'à 4 atomes de carbone, ou un atome d'hydrogène.

**20.** Composé selon les revendications 18 et 19, caractérisé par le fait que le groupe partant contient un atome d'azote.

**21.** Composé selon les revendications 18 et 19, caractérisé par le fait que le groupe partant contient un atome de soufre.

**22.** Composé selon les revendications 18 et 19 caractérisé par le fait que le groupe partant contient un atome de

sélénium.

**23.** Composé selon les revendications 18 et 19 caractérisé par le fait que le groupe partant contient un atome de tellure.

**24.** Composé selon les revendications 18 et 19 caractérisé par le fait que le groupe partant contient un atome de silicium.

**25.** Composé selon les revendications 18 et 19 caractérisé par le fait que le groupe partant contient un atome d'halogène.

**26.** Composé selon les revendications 18 et 19, caractérisé par le fait que le groupe partant contient un atome d'oxygène.

**27.** Composé selon les revendications 18, 19 et 25, caractérisé par le fait que le groupe partant est un atome d'halogène.

**28.** Composé selon les revendications 18, 19 et 26, caractérisé par le fait que le groupe partant est une fonction -OH.

**29.** Composé selon les revendications 18 et 19, caractérisé par le fait que le groupe partant est une fonction -COOH.

**30.** Composé selon les revendications 18 et 19, caractérisé par le fait que le groupe partant est une fonction -NH2.

**31.** Composé selon les revendications 18 et 19, caractérisé par le fait que le groupe partant est une fonction -CONH2.

**32.** Composé selon les revendications 18, 19 et 26, caractérisé par le fait que le groupe partant est une fonction -SO3C6H4pCH3.

**33.** Composé selon les revendications 18 et 21, caractérisé par le fait que le groupe partant est une fonction -SH.

**34.** Composé selon la revendication 18 et 19, caractérisé par le fait que le groupe partant est une fonction -CN.

**35.** Composé selon les revendications 18 et 24, caractérisé par le fait que le groupe partant est une fonction -Si(CH3) 3.

**36.** Composé selon les revendications 18 et 19, caractérisé par le fait que la fonction protégée de ladite molécule à protéger est une fonction alcool, thiol, acide, amine ou amide.

**37.** Composé selon la revendication 19, caractérisé par le fait que L'est -CO-.

**38.** Composé selon la revendication 19, caractérisé par le fait que L'est -O-CO-.

**39.** Composé selon la revendication 19, caractérisé par le fait que L'est -S-.

**40.** Composé selon la revendication 19, caractérisé par le fait que L'est -O-.

**41.** Composé selon la revendication 19, caractérisé par le fait que L'est -(CH2)m-O-où m est de 1 à 6.

**42.** Composé selon la revendication 19, caractérisé par le fait que L' est un lien direct.

**43.** Composé selon les revendications 18,19 et 36 à 42, caractérisé par le fait que la formule (I) répond à la formule (II)

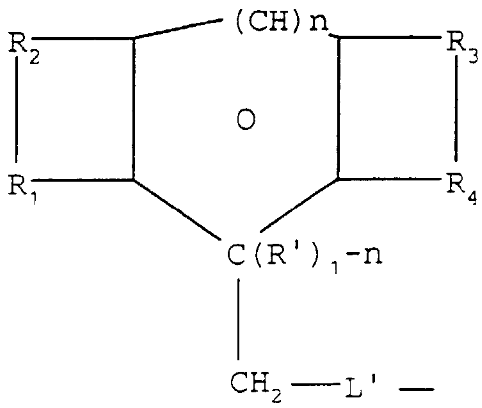

où n est égal à 0 ou 1;

O signifie que le cycle environnant est aromatique quand n est égal à 1 et non-aromatique quand n est égal à 0 ; R1 et R2, d'une part, et R3 et R4, d'autre part, sont choisis de façon à ce que chacun forme un système de cycles aromatiques fusionnés ensemble avec les atomes de carbone auxquels ils sont rattachés ; R' est un groupe alcoyl ou hydrogène ; et L'représente un lien direct ou un groupe capable de se lier à un groupe à protéger.

**44.** Composé selon la revendication 36 à 42, caractérisé par le fait que la formule (I) répond à la formule (III)/

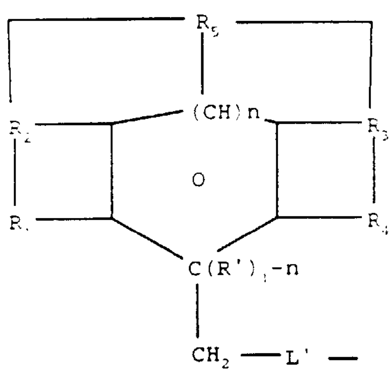

où n est égal à 0 ou 1;

O signifie que le cycle environnant est aromatique quand n est égal à 1 et non-aromatique quand n est égal à 0 ; R1 et R2, d'une part, et R3 et R4, d'autre part, sont choisis de façon à ce que chacun forme un système de cycles aromatiques fusionnés ensemble avec les atomes de carbone auxquels ils sont rattachés ; R' est un groupe alcoyl ou hydrogène ; et L' représente un lien direct ou un groupe capable de se lier à un groupe à protéger.
et où R5 est un groupe qui, ensemble avec les atomes auxquels il est rattaché, forme un ou plusieurs cycles supplémentaires.

**45.** Utilisation pour le procédé selon la revendication 1 d'un appareil comprenant pour utilisation successive ou simultanée :

a) une chambre remplie de matériau graphite; et
b) un nécessaire pour greffer sur une molécule un groupe protecteur de formule (I):

Ar-L-

où

Ar représente un système de cycles fusionnés substantiellement plan contenant au moins 4 cycles aromatiques, et
L représente un groupe contenant au moins un atome de carbone qui est capable de se lier à une fonction à protéger.

**46.** Utilisation selon la revendication 45, caractérisé par le fait que le matériau graphite est du graphite ou du charbon actif.

**47.** Utilisation selon les revendications 45 et 46, caractérisé par le fait qu 'il comprend également :

c) un système collecteur de fractions équipé d'un détecteur d'ultraviolets (UV), spectroscopique visible ou spectroscopique fluorescent.

## Patentansprüche

**1.** Verfahren, dadurch gekennzeichnet, daß es einen Verfahrensschritt umfaßt, bei dem ein Molekül, bei dem mindestens eine Funktion durch eine Gruppe der Formel (I)

Ar-L-

geschützt ist, physikalisch an ein kohlenstoffhaltiges Material gebunden wird, wobei:
Ar ein im wesentlichen ebenes System von miteinander verbundenen Ringen darstellt, das mindestens 4 aromatische Ringe enthält, und
L eine Gruppe darstellt, die mindestens ein Kohlenstoffatom enthält, das sich mit einer zu schützenden Funktion verbinden kann und
das kohlenstoffhaltige Material ausgewählt ist aus Graphit oder Materialien mit graphitisierten Oberflächen einschließlich Aktivkohle.

**2.** Verfahren nach Anspruch 1, dadurch gekennzeichnet, daß der Verfahrensschritt, bei dem die physikalische Bindung an ein kohlenstoffhaltiges Material erfolgt, in einem Durchleiten der Mischung von Verbindungen durch eine Kammer besteht, die mit dem kohlenstoffhaltigen Material gefüllt ist.

**3.** Verfahren nach Anspruch 1 oder 2, dadurch gekennzeichnet, daß es außerdem einen Verfahrensschritt umfaßt, bei dem mindestens eine Funktion in mindestens einer Verbindung mittels einer Gruppe der Formel (I) geschützt wird.

**4.** Verfahren nach den Ansprüchen 1 bis 3, dadurch gekennzeichnet, daß Ar mindestens 6 aromatische Ringe enthält.

**5.** Verfahren nach den Ansprüchen 1 bis 4, dadurch gekennzeichnet, daß die aromatischen Ringe sechseckig (hexagonal) sind.

**6.** Verfahren nach den Ansprüchen 1 bis 5, dadurch gekennzeichnet, daß Ar kein Heteroatom enthält.

**7.** Verfahren nach den Ansprüchen 1 bis 6, dadurch gekennzeichnet, daß die Formel (I) der folgenden Formel (II) entspricht

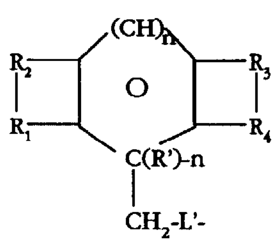

wobei:

n gleich 0 oder 1 ist;
O bedeutet, daß der umgebende Ring aromatisch ist, wenn n gleich 1 ist, und der umgebende Ring nichtaromatisch ist, wenn n gleich 0 ist; $R_1$ und $R_2$ einerseits und $R_3$ und $R_4$ andererseits derart ausgewählt sind, daß jeder der Reste gemeinsam mit den Kohlenstoffatomen, an die sie gebunden sind, ein System von verbundenen aromatischen Ringen bildet; R' eine Alkylgruppe oder ein Wasserstoffatom ist; und L' eine direkte Bindung darstellt oder eine Gruppe ist, die sich mit einer zu schützenden Gruppe verbinden kann.

**8.** Verfahren nach den Ansprüchen 1 bis 6, dadurch gekennzeichnet, daß die Formel (I) der Formel (III) entspricht

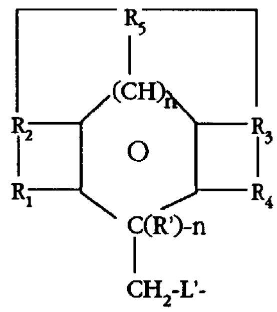

wobei:

n gleich 0 oder 1 ist;
O bedeutet, daß der umgebende Ring aromatisch ist, wenn n gleich 1 ist, und der umgebende Ring nichtaromatisch ist, wenn n gleich 0 ist; $R_1$ und $R_2$ einerseits und $R_3$ und $R_4$ andererseits derart ausgewählt sind, daß jeder der Reste zusammen mit den Kohlenstoffatomen, an die sie gebunden sind, ein System von verbundenen aromatischen Ringen bildet; R' eine Alkylgruppe oder ein Wasserstoffatom ist; und L' eine direkte Bindung darstellt oder eine Gruppe ist, die sich an eine zu schützende Gruppe binden kann; und $R_5$ eine Gruppe ist, die zusammen mit den Atomen, an die sie gebunden ist, ein oder mehrere weitere Ringe bildet.

**9.** Verfahren nach Anspruch 7 oder 8, dadurch gekennzeichnet, daß L' die Gruppe -CO-, -O-CO-, -S, -O-, $-(CH_2)_m-$O- ist, wobei m eine Zahl von 1 bis 6 ist oder einfach eine direkte Bindung darstellt.

**10.** Verfahren nach den Ansprüchen 1 bis 8, dadurch gekennzeichnet, daß die zu schützende Funktion des Moleküls eine Alkohol-, Thiol-, Säure-, Amin- oder Amidfunktion ist.

**11.** Verfahren nach den Ansprüchen 1 bis 10, dadurch gekennzeichnet, daß das zu schützende Molekül ein natürliches oder synthetisches Peptid oder eine Aminosäure ist.

**12.** Verwendung einer Gruppe der folgenden Formel (I)

Ar-L-

als Schutzgruppe, wobei:

Ar ein im wesentlichen ebenes System von miteinander verbundenen Ringen darstellt, das mindestens 4 aromatische Ringe enthält, und
L eine Gruppe darstellt, die mindestens ein Kohlenstoffatom enthält, das sich an eine zu schützende Gruppe binden kann.

**13.** Verwendung nach Anspruch 12, dadurch gekennzeichnet, daß Ar mindestens 6 aromatische Ringe enthält.

**14.** Verwendung nach Anspruch 13 oder 14, dadurch gekennzeichnet, daß die aromatischen Ringe sechseckig (hexagonal) sind.

**15.** Verwendung nach den Ansprüchen 12 bis 14, dadurch gekennzeichnet, daß Ar kein Heteroatom enthält.

**16.** Verwendung nach den Ansprüchen 12 bis 15, dadurch gekennzeichnet, daß die Formel (I) der Formel (II) entspricht

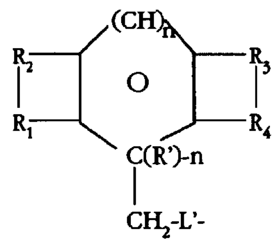

wobei:

n gleich 0 oder 1 ist;
O bedeutet, daß der umgebende Ring aromatisch ist, wenn n gleich 1 ist, und der umgebende Ring nichtaromatisch ist, wenn n gleich 0 ist; $R_1$ und $R_2$ einerseits und $R_3$ und $R_4$ andererseits derart ausgewählt sind, daß jeder der Reste gemeinsam mit den Kohlenstoffatomen, an die sie gebunden sind, ein System von verbundenen aromatischen Ringen bildet; R' eine Alkylgruppe oder ein Wasserstoffatom ist; und L' eine direkte Bindung darstellt oder eine Gruppe ist, die sich mit einer zu schützenden Gruppe verbinden kann.

**17.** Verwendung nach den Ansprüchen 12 bis 15, dadurch gekennzeichnet, daß die Formel (I) der Formel (III) entspricht

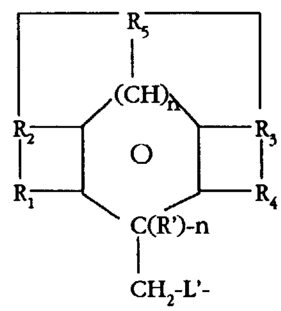

wobei:

n gleich 0 oder 1 ist;
O bedeutet, daß der umgebende Ring aromatisch ist, wenn n gleich 1 ist, und der umgebende Ring nichtaromatisch ist, wenn n gleich 0 ist; $R_1$ und $R_2$ einerseits und $R_3$ und $R_4$ andererseits derart ausgewählt sind, daß jeder der Reste zusammen mit den Kohlenstoffatomen, an die sie gebunden sind, ein System von verbundenen aromatischen Ringen bildet; R' eine Alkylgruppe oder ein Wasserstoffatom ist; und L' eine direkte Bindung darstellt oder eine Gruppe ist, die sich an eine zu schützende Gruppe binden kann; und $R_5$ eine Gruppe ist, die zusammen mit den Atomen, an die sie gebunden ist, ein oder mehrere weitere Ringe bildet.

**18.** Verbindung, verwendbar für die Durchführung des Verfahrens nach Anspruch 1, dadurch gekennzeichnet, daß sie eine Gruppe der Formel (I)

Ar-L-

enthält, wobei:

Ar ein im wesentlichen ebenes System von miteinander verbundenen Ringen darstellt, das mindestens 6 aromatische Ringe enthält, und

L eine Gruppe darstellt, die mindestens ein Kohlenstoffatom enthält, das sich mit einer zu schützenden Funktion verbinden kann und über die Gruppe L mit einer Abgangsgruppe (Austrittsgruppe) oder an ein zu schützendes Molekül gebunden ist.

**19.** Verbindung nach Anspruch 18, dadurch gekennzeichnet, daß L eine Gruppe CR'-L'- ist, wobei L' eine direkte Bindung oder eine Gruppe darstellt, die sich an eine zu schützende Gruppe binden kann, und R' eine Alkylgruppe vorzugsweise mit bis zu 4 Kohlenstoffatomen oder ein Wasserstoffatom ist.

**20.** Verbindung nach Anspruch 18 oder 19, dadurch gekennzeichnet, daß die Abgangsgruppe ein Stickstoffatom enthält.

**21.** Verbindung nach Anspruch 18 oder 19, dadurch gekennzeichnet, daß die Abgangsgruppe ein Schwefelatom enthält.

**22.** Verbindung nach Anspruch 18 oder 19, dadurch gekennzeichnet, daß die Abgangsgruppe ein Selenatom enthält.

**23.** Verbindung nach Anspruch 18 oder 19, dadurch gekennzeichnet, daß die Abgangsgruppe ein Telluratom enthält.

**24.** Verbindung nach Anspruch 18 oder 19, dadurch gekennzeichnet, daß die Abgangsgruppe ein Siliciumatom enthält.

**25.** Verbindung nach Anspruch 18 oder 19, dadurch gekennzeichnet, daß die Abgangsgruppe ein Halogenatom enthält.

**26.** Verbindung nach Anspruch 18 oder 19, dadurch gekennzeichnet, daß die Abgangsgruppe ein Sauerstoffatom enthält.

**27.** Verbindung nach den Ansprüchen 18, 19 und 25, dadurch gekennzeichnet, daß die Abgangsgruppe ein Halogenatom ist.

**28.** Verbindung nach den Ansprüchen 18, 19 und 26, dadurch gekennzeichnet, daß die Abgangsgruppe eine OH-Funktion ist.

**29.** Verbindung nach Anspruch 18 oder 19, dadurch gekennzeichnet, daß die Abgangsgruppe eine COOH-Funktion ist.

**30.** Verbindung nach Anspruch 18 oder 19, dadurch gekennzeichnet, daß die Abgangsgruppe eine $NH_2$-Funktion ist.

**31.** Verbindung nach Anspruch 18 oder 19, dadurch gekennzeichnet, daß die Abgangsgruppe eine $CONH_2$-Funktion ist.

**32.** Verbindung nach den Ansprüchen 18, 19 und 26, dadurch gekennzeichnet, daß die Abgangsgruppe eine $SO_3C_6H_4$-$pCH_3$-Gruppe ist.

**33.** Verbindung nach Anspruch 18 oder 21, dadurch gekennzeichnet, daß die Abgangsgruppe eine SH-Funktion ist.

**34.** Verbindung nach Anspruch 18 oder 19, dadurch gekennzeichnet, daß die Abgangsgruppe eine CN-Funktion ist.

**35.** Verbindung nach Anspruch 18 oder 24, dadurch gekennzeichnet, daß die Abgangsgruppe eine $Si(CH_3)_3$-Funktion ist.

**36.** Verbindung nach Anspruch 18 oder 19, dadurch gekennzeichnet, daß die geschützte Funktion des zu schützenden Moleküls eine Alkohol-, Thiol-, Säure-, Amin- oder Amidfunktion ist.

**37.** Verbindung nach Anspruch 19, dadurch gekennzeichnet, daß L' -CO- ist.

**38.** Verbindung nach Anspruch 19, dadurch gekennzeichnet, daß L' -O-CO- ist.

**39.** Verbindung nach Anspruch 19, dadurch gekennzeichnet, daß L' -S- ist.

**40.** Verbindung nach Anspruch 19, dadurch gekennzeichnet, daß L' -O- ist.

**41.** Verbindung nach Anspruch 19, dadurch gekennzeichnet, daß L' $-(CH_2)_m$-O-ist, wobei m eine Zahl von 1 bis 6 ist.

**42.** Verbindung nach Anspruch 19, dadurch gekennzeichnet, daß L' eine direkte Bindung ist.

**43.** Verbindung nach den Ansprüchen 18, 19 und 36 bis 42, dadurch gekennzeichnet, daß die Formel (I) der Formel (II) entspricht

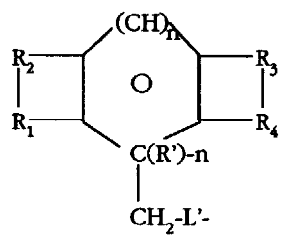

wobei:

n gleich 0 oder 1 ist;
O bedeutet, daß der umgebende Ring aromatisch ist, wenn n gleich 1 ist, und der umgebende Ring nichtaromatisch ist, wenn n gleich 0 ist; $R_1$ und $R_2$ einerseits und $R_3$ und $R_4$ andererseits derart ausgewählt sind, daß jeder der Reste gemeinsam mit den Kohlenstoffatomen, an die sie gebunden sind, ein System von verbundenen aromatischen Ringen bildet; R' eine Alkylgruppe oder ein Wasserstoffatom ist; und L' eine direkte Bindung darstellt oder eine Gruppe ist, die sich mit einer zu schützenden Gruppe verbinden kann.

**44.** Verbindung nach den Ansprüchen 36 bis 42, dadurch gekennzeichnet, daß die Formel (I) der Formel (III) entspricht

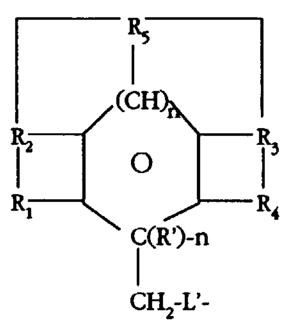

wobei:

n gleich 0 oder 1 ist;
O bedeutet, daß der umgebende Ring aromatisch ist, wenn n gleich 1 ist, und der umgebende Ring nichtaromatisch ist, wenn n gleich 0 ist; $R_1$ und $R_2$ einerseits und $R_3$ und $R_4$ andererseits derart ausgewählt sind, daß jeder der Reste zusammen mit den Kohlenstoffatomen, an die sie gebunden sind, ein System von verbundenen aromatischen Ringen bildet; R' eine Alkylgruppe oder ein Wasserstoffatom ist; und L' eine direkte Bindung darstellt oder eine Gruppe ist, die sich an eine zu schützende Gruppe binden kann; und $R_5$ eine Gruppe ist, die zusammen mit den Atomen, an die sie gebunden ist, ein oder mehrere weitere Ringe bildet.

**45.** Verwendung einer Apparatur für das Verfahren nach Anspruch 1, die zur aufeinanderfolgenden oder gleichzeitigen Verwendung umfaßt:

a) eine Kammer, die mit einem Graphitmaterial gefüllt ist; und
b)ein Gerät (Vorrichtung), um auf ein Molekül eine Schutzgruppe der Formel (I)

Ar-L-

zu pfropfen, wobei:

Ar ein im wesentlichen ebenes System von miteinander verbundenen Ringen darstellt, das mindestens 4 aromatische Ringe enthält, und
L eine Gruppe darstellt, die mindestens ein Kohlenstoffatom enthält, das sich an eine zu schützende Funktion binden kann.

**46.** Verwendung nach Anspruch 45, dadurch gekennzeichnet, daß das Graphitmaterial Graphit oder Aktivkohle ist.

**47.** Verwendung nach Anspruch 45 oder 46, dadurch gekennzeichnet, daß die Apparatur gleichermaßen enthält:

c) ein System zum Sammeln von Fraktionen, das mit einem Ultraviolettdetektor (UV-Detektor), einem Detektor für den spektroskopisch sichtbaren Bereich oder einem Detektor für den spektroskopischen fluoreszierenden Bereich ausgestattet ist.

## Claims

**1.** Process, characterized in that it comprises the stage of physically bonding, to a carbonaceous material, a molecule for which at least one functional group is protected by a group of formula (I):

Ar-L-

where

Ar represents a substantially planar system of fused rings comprising at least 4 aromatic rings, and
L represents a group comprising at least one carbon atom which is capable of being bonded to a functional group to be protected; and

where

the said carbonaceous material is chosen from graphite or those with graphitized surfaces, including active charcoal.

**2.** Process according to claim 1, characterized in that the stage of physically bonding to a carbonaceous material is a passage of the mixture of compounds through a chamber filled with the said carbonaceous material.

**3.** Process according to claims 1 and 2, characterized in that it additionally comprises the stage of protecting at least one functional group in at least one compound by means of a group of formula (I).

**4.** Process according to claims 1 to 3, characterized in that Ar comprises at least 6 aromatic rings.

**5.** Process according to claims 1 to 4, characterized in that the aromatic rings are hexagonal.

**6.** Process according to claims 1 to 5, characterized in that Ar does not contain any heteroatom.

**7.** Process according to claims 1 to 6, characterized in that the formula (I) corresponds to the following formula (II):

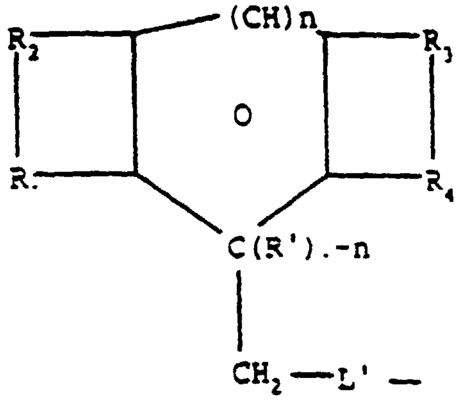

where

n is equal to 0 or 1;
O means that the surrounding ring is aromatic when n is equal to 1 and non-aromatic when n is equal to 0; $R_1$ and $R_2$, on the one hand, and $R_3$ and $R_4$, on the other hand, are chosen so that each forms a system of fused aromatic rings together with the carbon atoms to which they are attached; R' is an alkyl group or hydrogen; and L' represents a direct bond or a group capable of being bonded to a group to be protected.

**8.** Process according to claims 1 to 6, characterized in that the formula (I) corresponds to the formula (III):

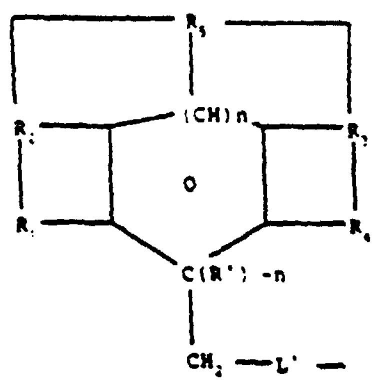

where

n is equal to 0 or 1;
O means that the surrounding ring is aromatic when n is equal to 1 and non-aromatic when n is equal to 0; $R_1$ and $R_2$, on the one hand, and $R_3$ and $R_4$, on the other hand, are chosen so that each forms a system of fused aromatic rings together with the carbon atoms to which they are attached; R' is an alkyl group or hydrogen;
and L' represents a direct bond or a group capable of being bonded to a group to be protected

and where

$R_5$ is a group which, together with the atoms to which it is attached, forms one or more additional rings.

**9.** Process according to claims 7 and 8, characterized in that L' is -CO-, -O-CO-, -S-, -O-, $-(CH_2)_m-O-$, where m is from 1 to 6, or simply a direct bond.

**10.** Process according to claims 1 to 8, characterized in that the functional group of the said molecule to be protected is an alcohol, thiol, acid, amine or amide group.

**11.** Process according to claims 1 to 10, characterized in that the said molecule to be protected is a natural or synthetic peptide or an amino acid.

**12.** Use as protective group of a group having the following formula (I): where

Ar represents a substantially planar system of fused rings comprising at least 4 aromatic rings and L represents a group comprising at least one carbon atom which is capable of being bonded to a group to be protected.

**13.** Use according to claim 12, characterized in that Ar comprises at least 6 aromatic rings.

**14.** Use according to claims 13 and 14, characterized in that the aromatic rings are hexagonal.

**15.** Use according to claims 12 to 14, characterized in that Ar does not comprise any heteroatom.

**16.** Use according to claims 12 to 15, characterized in that the formula (I) corresponds to the formula (II)

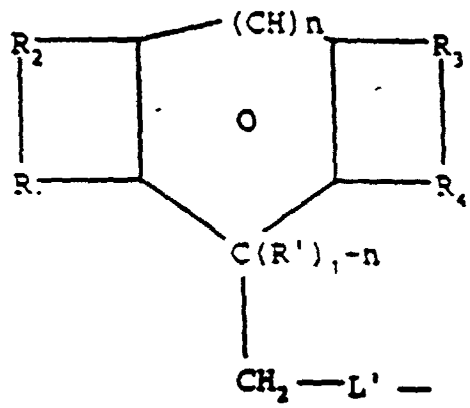

where

n is equal to 0 or 1;
O means that the surrounding ring is aromatic when n is equal to 1 and non-aromatic when n is equal to 0; $R_1$ and $R_2$, on the one hand, and $R_3$ and $R_4$, on the other hand, are chosen so that each forms a system of fused aromatic rings together with the carbon atoms to which they are attached; R' is an alkyl group or hydrogen; and L' represents a direct bond or a group capable of being bonded to a group to be protected.

**17.** Use according to claims 12 to 15, characterized in that the formula (I) corresponds to the formula (III):

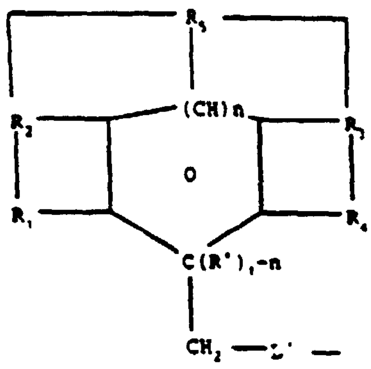

where

n is equal to 0 or 1;
O means that the surrounding ring is aromatic when n is equal to 1 and non-aromatic when n is equal to 0; $R_1$ and $R_2$, on the one hand, and $R_3$ and $R_4$, on the other hand, are chosen so that each forms a system of fused aromatic rings together with the carbon atoms to which they are attached; R' is an alkyl group or hydrogen; and L' represents a direct bond or a group capable of being bonded to a group to be protected

and where

$R_5$ is a group which, together with the atoms to which it is attached, forms one or more additional rings.

**18.** Compound of use in the implementation of the process according to claim 1, characterized in that it comprises a group of formula (I):

Ar-L-

where

Ar represents a substantially planar system of fused rings comprising at least 6 aromatic rings, and
L represents a group comprising at least one carbon atom, which is capable of being bonded to a functional group to be protected, attached, via the L group, to a leaving group or to a molecule to be protected.

**19.** Compound according to claim 18, characterized in that L is the -CR'-L'- group, where L' represents a direct bond or a group capable of being bonded to a group to be protected and R' is an alkyl group, preferably having up to 4 carbon atoms, or a hydrogen atom.

**20.** Compound according to claims 18 and 19, characterized in that the leaving group comprises a nitrogen atom.

**21.** Compound according to claims 18 and 19, characterized in that the leaving group comprises a sulphur atom.

**22.** Compound according to claims 18 and 19, characterized in that the leaving group comprises a selenium atom.

**23.** Compound according to claims 18 and 19, characterized in that the leaving group comprises a tellurium atom.

**24.** Compound according to claims 18 and 19, characterized in that the leaving group comprises a silicon atom.

**25.** Compound according to claims 18 and 19, characterized in that the leaving group comprises a halogen atom.

**26.** Compound according to claims 18 and 19, characterized in that the leaving group comprises an oxygen atom.

**27.** Compound according to claims 18, 19 and 25, characterized in that the leaving group is a halogen atom.

**28.** Compound according to claims 18, 19 and 26, characterized in that the leaving group is an -OH functional group.

**29.** Compound according to claims 18 and 19, characterized in that the leaving group is a -COOH functional group.

**30.** Compound according to claims 18 and 19, characterized in that the leaving group is an $-NH_2$ functional group.

**31.** Compound according to claims 18 and 19, characterized in that the leaving group is a $-CONH_2$ functional group.

**32.** Compound according to claims 18, 19 and 26, characterized in that the leaving group is an $-SO_3C_6H_4$-p-$CH_3$ functional group.

**33.** Compound according to claims 18 and 21, characterized in that the leaving group is an -SH functional group.

**34.** Compound according to claims 18 and 19, characterized in that the leaving group is a -CN functional group.

**35.** Compound according to claims 18 and 24, characterized in that the leaving group is an $-Si(CH_3)_3$ functional group.

**36.** Compound according to claims 18 and 19, characterized in that the protected functional group of the said molecule to be protected is an alcohol, thiol, acid, amine or amide functional group.

**37.** Compound according to claim 19, characterized in that L' is -CO-.

**38.** Compound according to claim 19, characterized in that L' is -O-CO-.

**39.** Compound according to claim 19, characterized in that L' is -S-.

**40.** Compound according to claim 19, characterized in that L' is -O-.

**41.** Compound according to claim 19, characterized in that L' is $-(CH_2)_m-O-$, where m is from 1 to 6.

**42.** Compound according to claim 19, characterized in that L' is a direct bond.

**43.** Compound according to claims 18, 19 and 36 to 42, characterized in that the formula (I) corresponds to the formula (II)

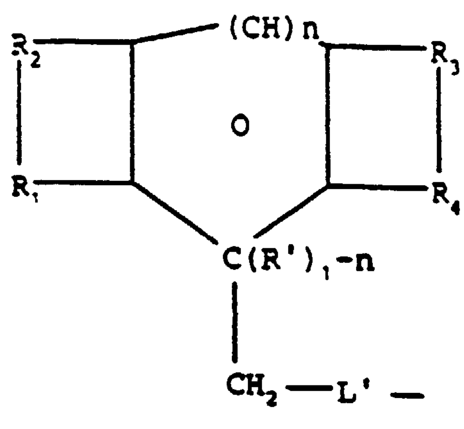

where

n is equal to 0 or 1;
O means that the surrounding ring is aromatic when n is equal to 1 and non-aromatic when n is equal to 0; $R_1$ and $R_2$, on the one hand, and $R_3$ and $R_4$, on the other hand, are chosen so that each forms a system of fused aromatic rings together with the carbon atoms to which they are attached; R' is an alkyl group or hydrogen; and L' represents a direct bond or a group capable of being bonded to a group to be protected.

**44.** Compound according to claims 36 to 42, characterized in that the formula (I) corresponds to the formula (III)

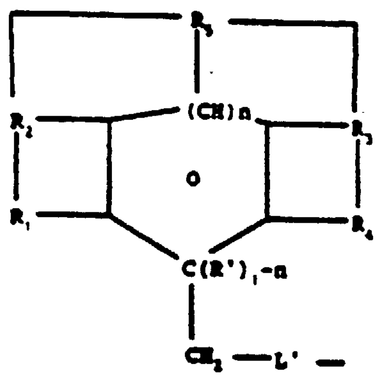

where

n is equal to 0 or 1;
O means that the surrounding ring is aromatic when n is equal to 1 and non-aromatic when n is equal to 0;
$R_1$ and $R_2$, on the one hand, and $R_3$ and $R_4$, on the other hand, are chosen so that each forms a system of fused aromatic rings together with the carbon atoms to which they are attached; R' is an alkyl group or hydrogen;
and L' represents a direct bond or a group capable of being bonded to a group to be protected

and where

$R_5$ is a group which, together with the atoms to which it is attached, forms one or more additional rings.

**45.** Use in the process according to claim 1 of a device comprising, for successive or simultaneous use:

a) a chamber filled with graphite material; and
b) an outfit for grafting, to a molecule, a protective group of formula (I):

Ar-L-

where

Ar represents a substantially planar system of fused rings comprising at least 4 aromatic rings, and
L represents a group comprising at least one carbon atom which is capable of being bonded to a functional group to be protected.

**46.** Use according to claim 45, characterized in that the graphite material is graphite or active charcoal

**47.** Use according to claims 45 and 46, characterized in that it also comprises:

c) a system for collecting fractions which is equipped with an ultraviolet (UV) radiation, visible spectroscopic or fluorescent spectroscopic detector.

FIGURE 1

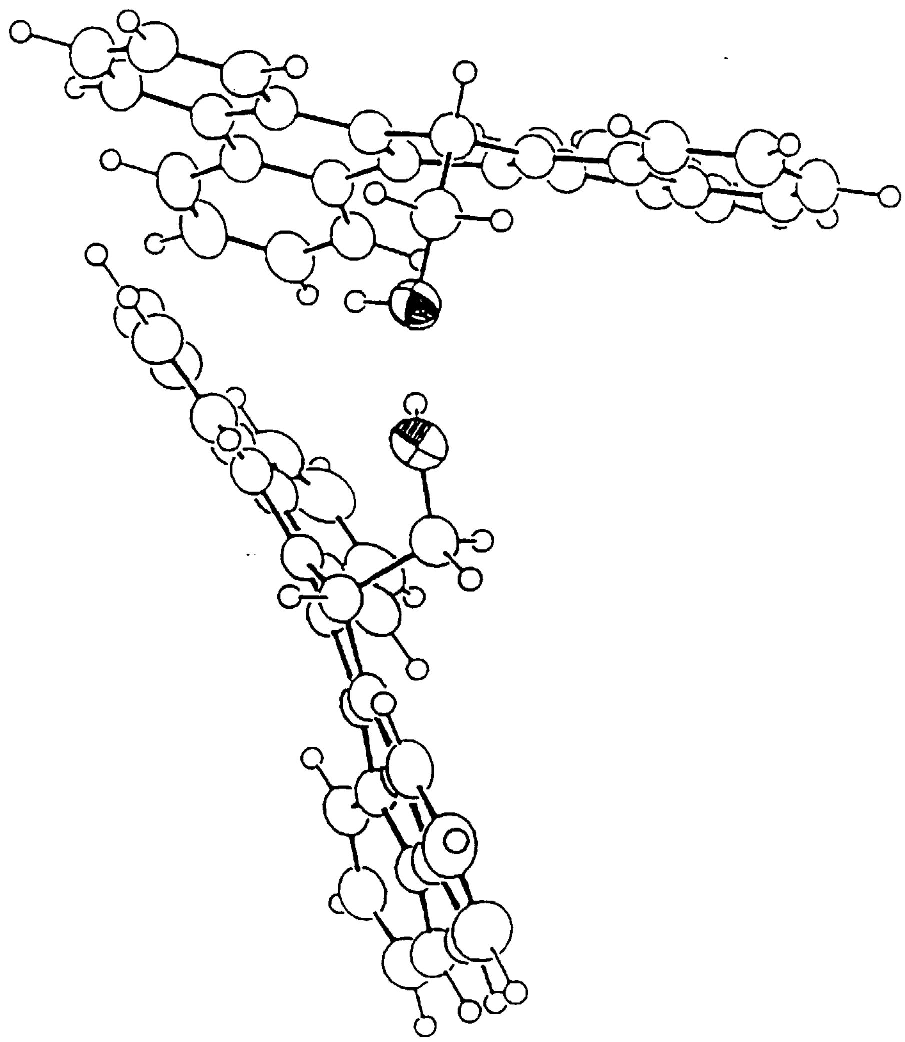

FIGURE 2